(19) Europäisches Patentamt
European Patent Office
Office européen des brevets

(11) Veröffentlichungsnummer: **0 311 135 B1**

(12) **EUROPÄISCHE PATENTSCHRIFT**

(45) Veröffentlichungstag der Patentschrift: **02.06.93**

(51) Int. Cl.[5]: **C07D 417/04**, C07D 513/04, C07D 413/04, C07D 403/04, C07D 471/04, C07D 487/04, A01N 43/90, A01N 43/78, A01N 43/52, A01N 43/60, A01N 43/80, //(C07D513/04, 285:00,209:00),(C07D513/04, 285:00,277:00),(C07D471/04, 235:00,221:00)

(21) Anmeldenummer: **88116762.1**

(22) Anmeldetag: **10.10.88**

Die Akte enthält technische Angaben, die nach dem Eingang der Anmeldung eingereicht wurden und die nicht in dieser Patentschrift enthalten sind.

(54) **Heterocyclisch substituierte Azole und Azine, Verfahren zu ihrer Herstellung und ihre Verwendung als Mittel mit herbizider Wirkung.**

(30) Priorität: **09.10.87 DE 3734745**

(43) Veröffentlichungstag der Anmeldung: **12.04.89 Patentblatt 89/15**

(45) Bekanntmachung des Hinweises auf die Patenterteilung: **02.06.93 Patentblatt 93/22**

(84) Benannte Vertragsstaaten: **AT BE CH DE ES FR GB GR IT LI LU NL SE**

(56) Entgegenhaltungen:
**EP-A- 0 176 101**
**EP-A- 0 177 032**
**EP-A- 0 218 972**
**EP-A- 0 230 874**
**EP-A- 0 235 567**
**EP-A- 0 255 601**
**EP-A- 0 258 773**
**FR-A- 1 570 220**
**US-A- 4 755 217**

(73) Patentinhaber: **SCHERING AKTIENGESELL-SCHAFT Berlin und Bergkamen**
**Müllerstrasse 170/178 Postfach 65 03 11**
**W-1000 Berlin 65(DE)**

(72) Erfinder: **Ganzer, Michael, Dr.**
**Dannenwalder Weg 149**
**W-1000 Berlin 26(DE)**
Erfinder: **Franke, Wilfried, Dr.**
**Spiessergasse 6b**
**W-1000 Berlin 27(DE)**
Erfinder: **Dorfmeister, Gabrielle, Dr.**
**Heiligenseestrasse 70**
**W-1000 Berlin 27(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 311 135 B1

**CHEMICAL ABSTRACTS, Band 96, Nr. 17, 26. April 1082, Columbus, Ohio, US; DOMAGALINA, EUGENIA et al.: "5-, 6-, 7-Succinimidobenzoxazolin-2-ones." Seite 748, Spalte 2, Zusammenfassung-Nr. 142 741y**

**CHEMICAL ABSTRACTS, Band 109, Nr. 1, 4. Juli 1988, Columbus, Ohio, US; TAKEMOTO, KAZUKI: "A process for the preparation of 6-fluoro-7-(3,4,5,6-tetrahydrophthalimido)-1,2-,3,4-tetrahydroquinoxalin-2-one as an intermediate for agrochemicals." Seite 618, Spalte 2, Zusammenfassung-Nr. 6 512j**

**CHEMICAL ABSTRACTS, Vol. 107, Nr. 23, 7. December 1987, Columbus, Ohio, USA HAGA, TORU et al.:"Preparation of 2-(6-fluoro-2-oxobenzothiazol-5-yl)tetahydroinidazol [1,5-a] pyridine-1, 3-dione derivatives as herbicides." Page 591,column 2, abstract nr. 217620q**

Erfinder: **Johann, Gerhard, Dr.**
**Hermsdorfer Damm 147**
**W-1000 Berlin 28(DE)**
Erfinder: **Arndt, Friedrich, Dr.**
**Mühlenfeldstrasse 10**
**W-1000 Berlin 28(DE)**
Erfinder: **Rees, Richard, Dr.**
**Speerweg 8**
**W-1000 Berlin 28(DE)**

## Beschreibung

Die Erfindung betrifft neue substituierte Azole und Azine, Verfahren zu ihrer Herstellung und ihre Verwendung als Mittel mit herbizider Wirkung.

Es ist bereits bekannt, daß bestimmte Triazolopyridazine und Benzothiazolylazolidine herbizide Eigenschaften besitzen (EP-Anmeldungen 0 176 101 und 0 230 874). Bei diesen bekannten Verbindungen ist die Herbizidwirkung jedoch nicht in allen Fällen ausreichend oder es treten Selektivitätsprobleme in wichtigen landwirtschaftlichen Kulturen auf.

Aufgabe der vorliegenden Erfindung ist daher die Bereitstellung von neuen Verbindungen, die diese Nachteile nicht aufweisen und in ihren biologischen Eigenschaften den bisher bekannten Verbindungen überlegen sind.

Es wurde nun gefunden, daß substituierte Azole und Azine der allgemeinen Formel I

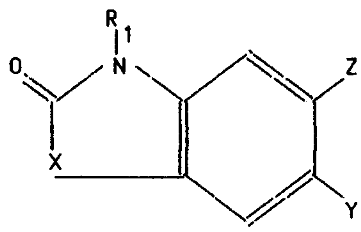

(I),

in der

| | |
|---|---|
| $R_1$ | ein Wasserstoffatom, eine $C_1$-$C_5$-Alkylgruppe, eine $C_3$-$C_5$-Alkenylgruppe, eine $C_3$-$C_5$-Alkinylgruppe, eine Halogen-$C_1$-$C_4$-alkylgruppe, eine Halogen-$C_3$-$C_5$-alkenylgruppe, eine Halogen-$C_3$-$C_5$-alkinylgruppe oder eine Cyano-$C_1$-$C_3$-alkylgruppe, |
| Y | ein Wasserstoff- oder Fluoratom, |
| X | eine der Gruppen $(CR_2R_3)_n$-W oder $(CR_2)=V$, wobei V beziehungsweise W direkt am Phenylkern gebunden sind, |
| n | 0 oder 1, |
| W | eine der Gruppen $CR_4R_5$ oder $NR_6$ oder ein Sauerstoff- oder Schwefelatom, mit der Einschränkung, daß, wenn Z = $Z_1$ und n = 1 bedeuten, W nicht Sauerstoff ist |
| V | eine Gruppe $CR_4$ oder ein Stickstoffatom, |
| $R_2$, $R_3$, $R_4$ und $R_5$ | gleich oder verschieden jeweils ein Wasserstoff- oder Halogenatom oder eine $C_1$-$C_3$-Alkyl- oder Halogen-$C_1$-$C_3$-alkylgruppe, |
| $R_6$ | ein Wasserstoffatom, |
| Z | einen heterocyclischen Rest der Formeln $Z_1$ und $Z_3$ bis $Z_8$ |

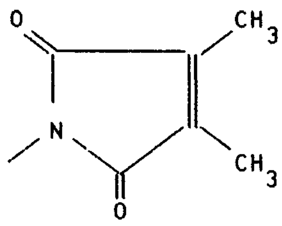

,

$Z_1$

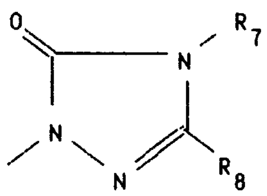

,

$Z_3$

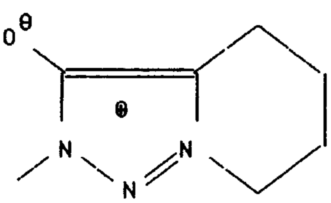

,

$Z_5$

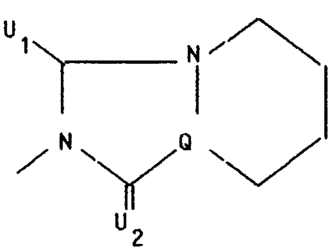

,

$Z_6$

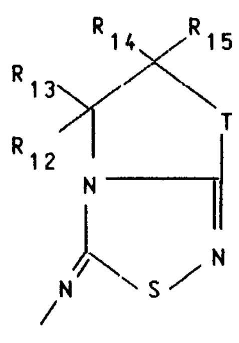

$Z_7$

oder

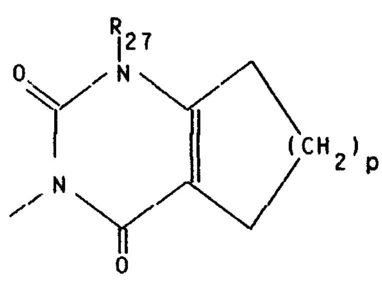

.

$Z_8$

| | |
|---|---|
| Q | die Gruppe CH, mit der Einschränkung, daß wenn n = 0 bedeutet, W nicht Schwefel ist, |
| $U_1$ | ein Sauerstoff- oder Schwefelatom, |
| $U_2$ | ein Sauerstoffatom, |
| $R_7$ und $R_8$ | gemeinsam mit den benachbarten Stickstoff- und Kohlenstoffatomen einen heterocyclischen 5- bis 7-gliedrigen Ring bildet, der gesättigt oder ungesättigt ist, |
| T | die Gruppe D-E, |

| | |
|---|---|
| D | eine Gruppe $(CR_{16}R_{17})_m$, |
| E | ein Schwefelatom oder die Gruppe $CR_{19}R_{20}$, |
| m | = 0, |
| p | = 2, |
| $R_{12}$, $R_{13}$, $R_{14}$, $R_{15}$, $R_{16}$, $R_{17}$, $R_{18}$, $R_{19}$ und $R_{20}$ | ein Wasserstoffatom oder eine $C_1$-$C_4$-Alkylgruppe und |
| $R_{27}$ | ein Wasserstoffatom oder eine $C_1$-$C_5$-Alkylgruppe bedeutet, |

überraschenderweise eine hervorragende Verträglichkeit für Kulturpflanzen bei gleichzeitig interessanter herbizider Wirkung zeigen.

Der Ausdruck Halogen steht für Fluor, Chlor, Brom oder Jod. Die Bezeichnung Halogenalkyl besagt, daß ein oder mehrere Wasserstoffatome des Alkylrestes durch Halogen ersetzt sind.

Als Beispiel für heterocyclische Ringe seien genannt: Pyrrol, Oxazol, Thiazol, Imidazol, Pyridin, Oxazin, Thiazin, Pyrimidin, Pyrazin, Triazin, Oxadiazin und Thiadiazin sowie ihre Di-, Tetra- oder gegebenenfalls Hexahydroderivate.

Die erfindungsgemäßen Verbindungen der allgemeinen Formel I lassen sich herstellen, indem man

A) falls Z einen 3,4-Dimethyl-3-pyrrolin-2,5-dion-1-yl-Rest darstellt, ein Anilin der allgemeinen Formel II

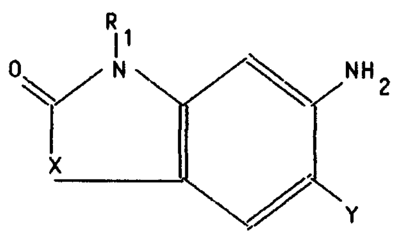

(II),

in der $R_1$, X und Y die unter der allgemeinen Formel I genannten Bedeutungen haben, mit 2,2'-Dimethylmaleinsäureanhydrid umsetzt,

B) falls Z einen 4,5,6,7-Tetrahydro-2H-1,2,3-triazolo[3,4-a]pyridin-8-ium-3-olat-2-yl-Rest darstellt, eine Verbindung der allgemeinen Formel II

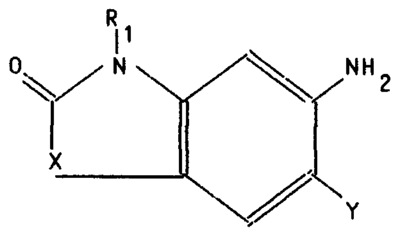

(II),

in der $R_1$, X und Y die unter der allgemeinen Formel I angegebenen Bedeutungen haben, mit salpetriger Säure diazotiert, mit Piperidin-2-carbonsäure umsetzt und mit Hilfe von dehydratisierenden Mitteln cyclisiert,

C) falls Z einen 2,3,5,6,7,8-Hexahydro-1H-imidazo[1,5-a]pyridin-1,3-dion-2-yl- oder einen 2,3,5,6,7,8-Hexahydro-1H-1,3,4-triazolo[1,2-a]pyridazin-1,3-dion-2-yl-Rest darstellt, eine Verbindung der allgemeinen Formel II

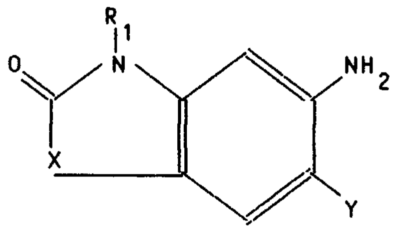

(II),

in der $R_1$, X und Y die unter der allgemeinen Formel I genannten Bedeutungen haben, mit einer Verbindung der allgemeinen Formel V oder VI

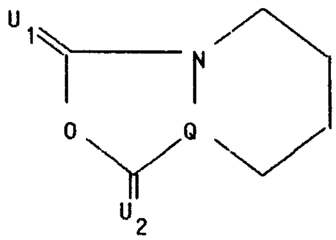

(V)

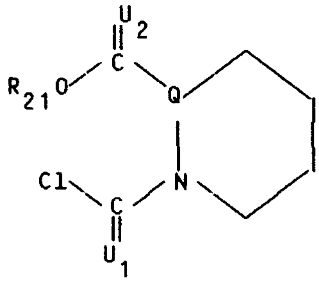

(VI),

in denen Q, $U_1$ und $U_2$ die unter der allgemeinen Formel I genannten Bedeutungen haben und $R_{21}$ einen $C_1$-$C_4$-Alkylrest darstellt, umsetzt,

D) falls Z einen 2,3,5,6,7,8-Hexahydro-1H-imidazo[1,5-a]pyridin-1,3-dion-2-yl- oder einen 2,3,5,6,7,8-Hexahydro-1H-1,3,4-triazolo[1,2-a]pyridazin-1,3-dion-2-yl-Rest darstellt, eine Verbindung der allgemeinen Formel III

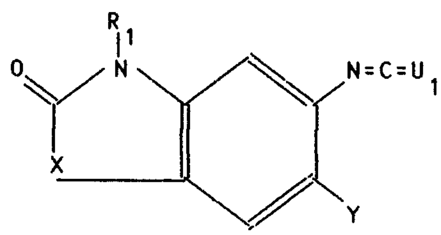

(III),

in der $R_1$, $U_1$, X und Y die unter der allgemeinen Formel I genannten Bedeutungen haben, mit einer Verbindung der allgemeinen Formel VII

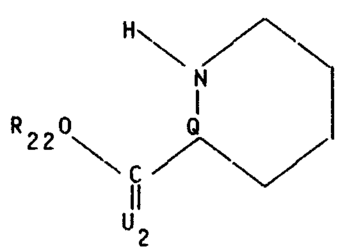

(VII)

in der Q und $U_2$ die unter der allgemeinen Formel I genannten Bedeutungen haben und $R_{22}$ einen $C_1$-$C_4$-Alkylrest darstellt, zur Reaktion bringt,

E) falls Z einen Rest der allgemeinen Formel

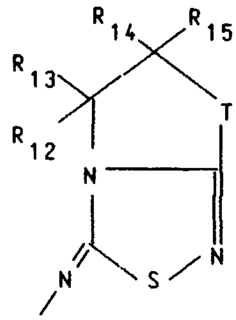

darstellt, in der $R_{12}$, $R_{13}$, $R_{14}$, $R_{15}$ und T die unter der allgemeinen Formel I genannten Bedeutungen haben, eine Verbindung der allgemeinen Formel IIIa

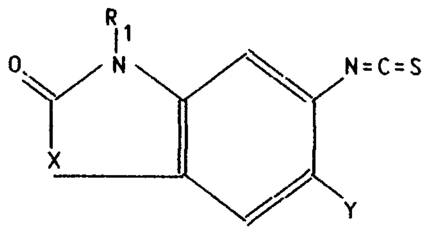

(IIIa),

in der $R_1$, X und Y die unter der allgemeinen Formel I genannten Bedeutungen haben, mit einer Verbindung der allgemeinen Formel VIII

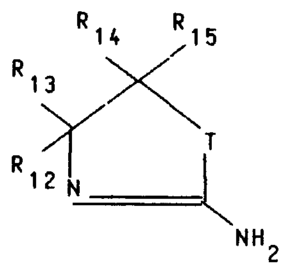

(VIII).

in der $R_{12}$, $R_{13}$, $R_{14}$, $R_{15}$ und T die unter der allgemeinen Formel I genannten Bedeutungen haben, umsetzt und in Gegenwart eines Oxidationsmittels zum Thiadiazolring cyclisiert,
I) falls Z einen 2H-1,2,4-Triazolin-3-on-2-yl-Rest darstellt, eine Verbindung der allgemeinen Formel IV

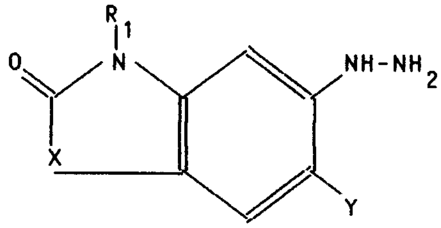

(IV).

in der $R_1$, X und Y die in der allgemeinen Formel I genannten Bedeutungen haben, mit einer Verbindung der allgemeinen Formel XI

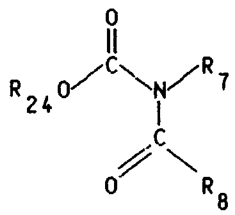

(XI),

worin $R_7$ und $R_8$ die unter der allgemeinen Formel I genannten Bedeutungen haben und und $R_{24}$ einen $C_1$-$C_4$-Alkylrest darstellt, umsetzt,

K) falls Z einen 5,6,7,8-Tetrahydrochinazolin-2,4(1H,3H)-dion-3-yl-Rest oder einen 1,2,4,5,6,7-Hexahydro-2,4-dioxo-3H-cyclopentapyrimidin-3-yl-Rest darstellt, eine Verbindung der allgemeinen Formel XXII

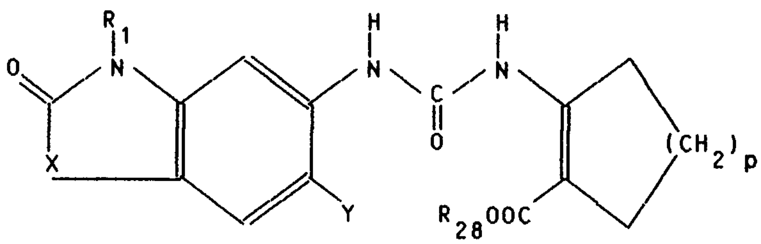

(XXII),

in der $R_1$, X, Y und p, die unter der allgemeinen Formel I angegebenen Bedeutungen haben und $R_{28}$ einen $C_1$-$C_4$-Alkylrest darstellt, unter basischen Bedingungen cyclisiert und gegebenenfalls mit einer Verbindung der allgemeinen Formel XXIII

$R_{27}$ - B (XXIII),

in der $R_{27}$ die unter der allgemeinen Formel I angegebene Bedeutung hat, aber nicht ein Wasserstoffatom darstellt, und B ein Halogenatom oder die p-Toluolsulfonyloxygruppe bedeutet, gegebenenfalls unter Verwendung eines säurebindenden Mittels umsetzt.

Die Umsetzung gemäß der Verfahrensvariante A) wird zweckmäßig bei 20 °C bis 200 °C gegebenenfalls in einem geeigneten Lösungsmittel durchgeführt, wobei 2,2'-Dimethylmaleinsäureanhydrid in einem Molverhältnis von 1 bis 3 Äquivalenten zu 1 Äquivalent Amin der Formel II eingesetzt wird. Die Reaktionszeit beträgt 1 bis 24 Stunden. Es ist zweckmäßig, die Reaktion in Gegenwart einer Säure, wie beispielsweise Essigsäure, durchzuführen, zum Beispiel indem man in Essigsäure als Lösungsmittel arbeitet. Es ist aber auch möglich, die beiden Reaktionspartner unter Verwendung eines inerten Lösungsmittels, wie zum Beispiel Dichlormethan oder Dimethylsulfoxid, zur Reaktion zu bringen und das intermediär entstandene Additionsprodukt mit Säureanhydriden, wie zum Beispiel Acetanhydrid, zu cyclisieren.

Die Umsetzung gemäß Verfahrensvariante B) erfolgt im allgemeinen in drei Stufen ohne zwischenzeitliche Aufreinigung der Produkte gemäß dem folgenden Formelschema:

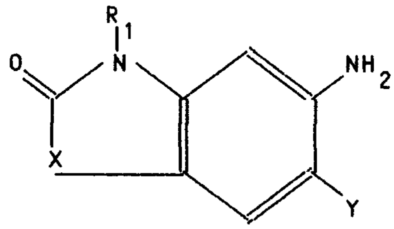

(II),

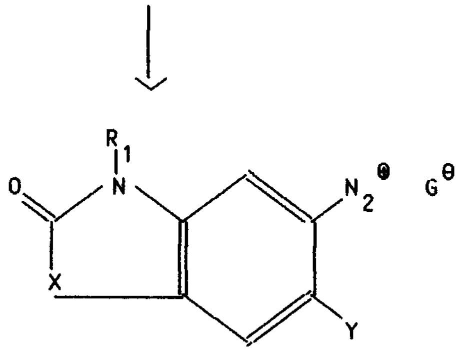

(XIV),

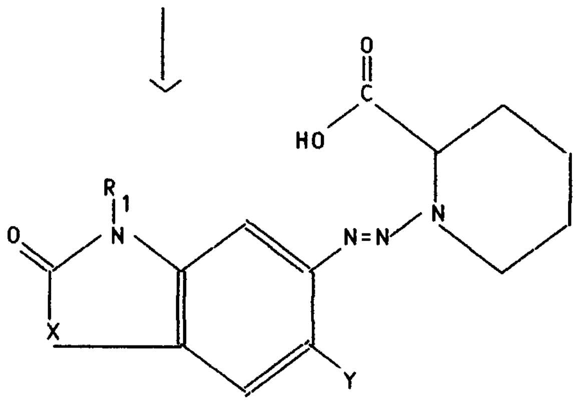

(XV)

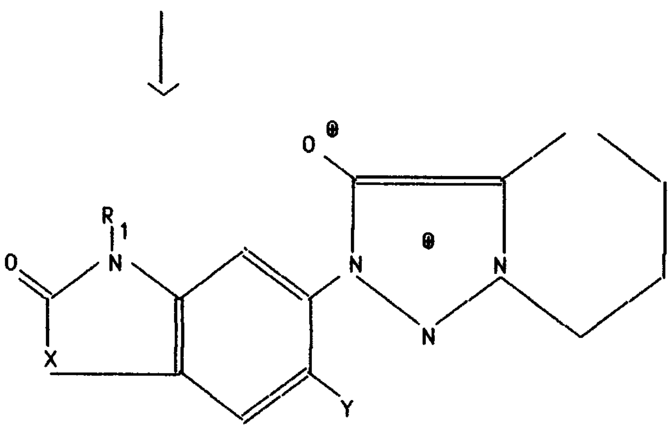

(Ia)

Die Aniline der allgemeinen Formel II, in der $R_1$, X und Y die unter der allgemeinen Formel I angegebenen Bedeutungen haben, werden nach den üblichen Verfahren in wäßriger, saurer Suspension bei einer Temperatur von -20° bis +10°C mit Natriumnitrit diazotiert, wobei die Reaktionsdauer 0,5 bis 3 Stunden beträgt.

Die Verbindungen der allgemeinen Formel XIV, in der G ein Halogenatom, insbesondere ein Chloratom, andere anorganische Reste, wie Hydrogensulfat, oder organische Reste, wie Acetat, darstellt, werden anschließend bei einer Temperatur von -20° bis +20 °C mit Piperidin-2-carbonsäure und säurebindenden Mitteln, wie zum Beispiel Trialkylaminen, umgesetzt.

Die Verbindungen der allgemeinen Formel XV können mit Säureanhydriden, wie zum Beispiel Acetanhydrid, unter Verwendung einer Base, wie zum Beispiel Pyridin, in einem inerten Lösungsmittel, wie zum Beispiel Ether, zu den Verbindungen der Formel Ia cyclisiert werden.

Die Umsetzung gemäß Verfahrensvariante C) wird zweckmäßig bei einer Temperatur oberhalb 20 °C, zum Beispiel bei 100 °C oder bei Rückflußtemperatur der Reaktionsmischung durchgeführt. Falls der Reaktionspartner ein Anhydrid der allgemeinen Formel V ist, wird die Reaktion zweckmäßig in Gegenwart einer Säure wie Essigsäure durchgeführt, zum Beispiel indem man in Essigsäure als Lösungsmittel arbeitet. Es ist aber auch möglich, die beiden Reaktionspartner ohne oder unter Verwendung eines inerten Lösungsmittels, wie zum Beispiel Dichlormethan oder Dimethylsulfoxid, zur Reaktion zu bringen und das intermediär entstandene Additionsprodukt mit Säureanhydriden, wie zum Beispiel Acetanhydrid, zu cyclisieren.

Die Umsetzung gemäß Verfahrensvariante D) erfolgt in einem inerten Lösungsmittel bei Temperaturen zwischen 20 °C und 150 °C, bevorzugt bei der Siedetemperatur des Lösungsmittels. Geeignete Lösungsmittel sind zum Beispiel aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlorethan, Chlorbenzol, sowie Ether, wie Diethyl- und Di-n-butylether, Methyl-tert.-butylether, Tetrahydrofuran, Dioxan, Ketone, wie beispielsweise Aceton, Methylethylketon, Methylisopropylketon, ferner Nitrile, wie Acetonitril und Propionitril, Sulfoxide, wie Dimethylsulfoxid, oder Sulfolan.

Die als Ausgangsmaterial verwendeten Isocyanate beziehungsweise Isothiocyanate der allgemeinen Formel III lassen sich aus den Anilinen der allgemeinen Formel II, in der $R_1$, X und Y die unter der allgemeinen Formel I genannten Bedeutungen haben, nach an sich bekannten Verfahren mit Phosgen oder Thiophosgen, beziehungsweise deren reaktiven Derivaten leicht herstellen.

Die Umsetzung gemäß Verfahrensvariante E) erfolgt in einem inerten Lösungsmittel, wie zum Beispiel Ether, Methylenchlorid, Chloroform oder Ethylacetat, bei einer Temperatur von -50 °C bis +50 °C durch Umsetzung einer Verbindung der allgemeinen Formel IIIa, in der $R_1$, X und Y die unter der allgemeinen Formel I genannten Bedeutungen haben, mit einer Verbindung der allgemeinen Formel VIII, in der $R_{12}$, $R_{13}$, $R_{14}$, $R_{15}$ und T die unter der allgemeinen Formel I genannten Bedeutungen haben. Die Reaktionszeit beträgt 0,5 bis 10 Stunden. Die erhaltene Verbindung der allgemeinen Formel XVI

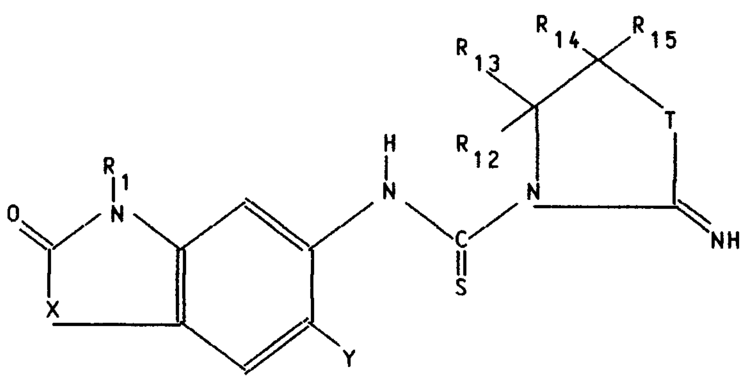

(XVI)

ist thermisch instabil und wird deshalb vorzugsweise ohne Isolierung in die nächste Reaktion eingesetzt.

Die Ringbildung wird unter Verwendung eines Oxidationsmittels in einem organischen Lösungsmittel durchgeführt. Als organisches Lösungsmittel kommt ein inertes Lösungsmittel, wie Methylenchlorid, Chloroform, N,N-Dimethylformamid und Ethylacetat, in Frage. Die Kondensationsreaktion unter Ringbildung kann in Gegenwart von wirksamen Säureakzeptoren, je nach Art des Oxidationsmittels, durchgeführt werden. Als Säureakzeptoren kommen organische Basen, wie Triethylamin, Pyridin, Dimethylanilin, anorganische Basen, wie Natriumhydroxid und Natriumcarbonat, in Frage. Als Oxidationsmittel werden Brom, Chlor, Natriumhypochlorid und andere eingesetzt. Steht mindestens ein Substituent R für die Hydroxygruppe, so wird als Oxidationsmittel Jod bevorzugt.

Die Umsetzung gemäß Verfahrensvariante I) wird zweckmäßig unter Katalyse in einem geeigneten Lösungsmittel durchgeführt. Die Reaktionstemperaturen liegen zwischen Raumtemperatur und 150 °C, bevorzugt bei Rückflußtemperatur des Reaktionsgemisches. Als geeignete Lösungsmittel seien beispielsweise genannt Dimethylsulfoxid, Halogenkohlenwasserstoffe, wie zum Beispiel Methylenchlorid und Chloroform, aromatische Kohlenwasserstoffe, wie zum Beispiel Benzol, Toluol, Xylol, Chlorbenzol und Dichlorbenzol, sowie andere gegenüber den Reaktionspartnern inerte Lösungsmittel, wie zum Beispiel Diethylether,

Tetrahydrofuran oder Dimethylformamid.

Als Katalysatoren können Säuren wie Essigsäure oder Schwefelsäure, aber auch saure Ionentauscher verwendet werden.

Die Umsetzung gemäß der Verfahrensvariante K) wird zweckmäßigerweise in einem geeigneten Lösungsmittel unter Zugabe einer Base, wie zum Beispiel einem Alkalihydroxid oder -alkoholat, Natriumhydrid oder Triethylamin, gegebenenfalls in einem Zweiphasensystem unter Zugabe eines Phasentransferkatalysators und bei Temperaturen zwischen 0 °C und 150 °C, bevorzugt bei Rückflußtemperatur des Lösungsmittels, durchgeführt.

Die Ausgangsverbindungen der Formel XXII werden gemäß dem folgendem Formelschema erhalten:

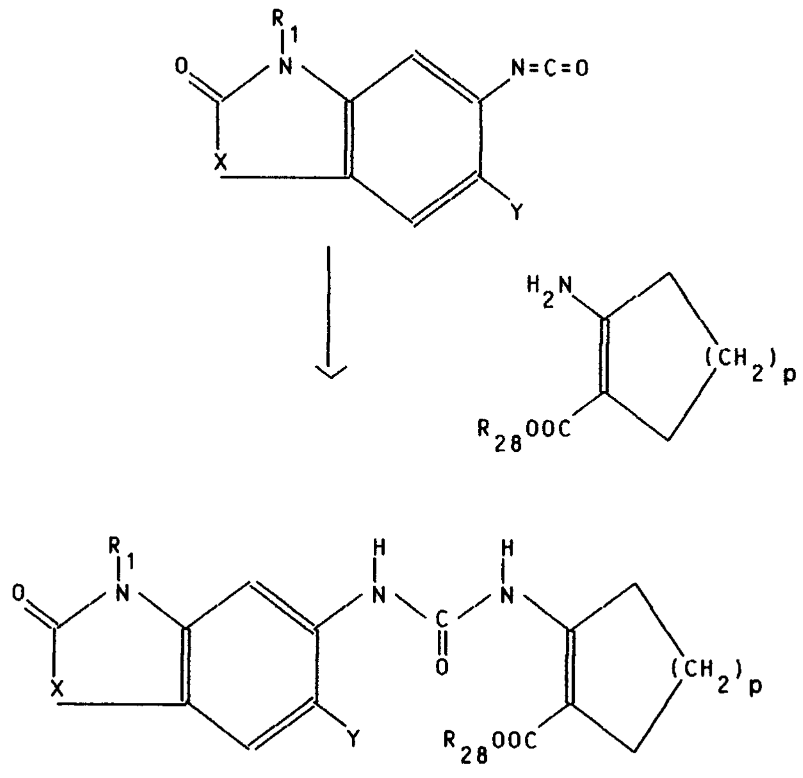

(IIIb)

(XXVI)

(XXII)

Die Umsetzung von Verbindungen der Formel IIIb, in der $R_1$, X und Y die unter der allgemeinen Formel I angegebenen Bedeutungen haben, mit Enaminoestern der Formel XXVI, in der p 1 oder 2 und $R_{28}$ eine $C_1$-$C_4$-Alkylgruppe bedeutet, erfolgt zweckmäßigerweise in einem inerten Lösungsmittel, wie zum Beispiel Dioxan. Die Reaktionstemperaturen liegen zwischen Raumtemperatur und 200 °C, bevorzugt bei der Siedetemperatur des Lösungsmittels.

Überlicherweise setzt man die Ausgangsstoffe in stöchiometrischem Verhältnis ein. Ein Überschuß des einen oder anderen kann in Einzelfällen aber durchaus vorteilhaft sein.

Die nach oben genannten Verfahren hergestellten erfindungsgemäßen Verbindungen können nach den üblichen Methoden aus dem Reaktionsgemisch isoliert werden, beispielsweise durch Abdestillieren des eingesetzten Lösungsmittels bei normalem oder vermindertem Druck oder durch Extraktion.

Ein erhöhter Reinheitsgrad kann in der Regel durch säulenchromatographische Aufreinigung sowie durch fraktionierte Destillation erhalten werden.

Die erfindungsgemäßen Verbindungen stellen in der Regel kristalline oder zähflüssige Substanzen dar, die zum Teil gut löslich in halogenierten Kohlenwasserstoffen, wie Chloroform, Sulfoxiden, wie Dimethylsulfoxid, oder Estern, wie Essigester, sind.

Die Ausgangsverbindungen der allgemeinen Formel III und IV sind weitgehend neu, lassen sich aber analog zu an sich bekannten Verfahren herstellen.

Die erfindungsgemäßen Wirkstoffe zeigen eine gute herbizide Wirkung bei breitblättrigen Unkräutern und Gräsern. Ein selektiver Einsatz der erfindungsgemäßen Wirkstoffe ist in verschiedenen Kulturen möglich, zum Beispiel in Raps, Rüben, Sojabohnen, Baumwolle, Reis, Gerste, Weizen und anderen Getreidearten. Dabei sind einzelne Wirkstoffe als Selektivherbizide in Rüben, Baumwolle, Soja und Getreide

besonders geeignet. Ebenso können die Verbindungen zur Unkrautbekämpfung in Dauerkulturen, zum Beispiel Forst-, Ziegerhölz-, Obst-, Wein-, Citrus-, Nuß-, Bananen-, Kaffee-, Tee-, Gummi-, Ölpalm-, Kakao-, Beerenfrucht- und Hopfenanlagen, und zur selektiven Unkrautbekämpfung in einjährigen Kulturen eingesetzt werden.

Die erfindungsgemäßen Wirkstoffe können zum Beispiel bei den folgenden Pflanzengattungen verwendet werden:
Dikotyle Unkräuter der Gattungen Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea und Chrysanthemum. Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Monocharia, Fimbristylis, Eleocharis, Ischaemum und Apera.

Die Aufwandmengen schwanken je nach Anwendungsart im Vor- oder Nachauflauf in Grenzen zwischen 0,05 bis 5 kg/ha.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden. Beispielsweise eignen sich als herbizid wirksame Mischungspartner diejenigen Wirkstoffe, die in Weed Abstracts, Vol. 35, No.5, 1986, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acylphosphatidylethanolaminen, Phosphatidylinosit, Phosphatidylserin, Lysolecithin und Phosphatidylglycerol.

Zweckmäßig werden die gekennzeichneten Wirkstoffe oder deren Mischungen in Form von Zubereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe, wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahrenist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

A) **Spritzpulver**

20 Gewichtsprozent Wirkstoff
35 Gewichtsprozent Bentonit

35 Gewichtsprozent Kieselsäure
8 Gewichtsprozent Calciumsalz der Ligninsulfonsäure
2 Gewichtsprozent Natriumsalz des N-Methyl-N-oleyl-taurins

B) **Paste**

45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtsprozent Spindelöl
10 Gewichtsprozent Polyethylenglycol
23 Gewichtsprozent Wasser

C) **Emulsionskonzentrat**

20 Gewichtsprozent Wirkstoff
75 Gewichtsprozent Isophoron
5 Gewichtsprozent einer Mischung aus dem Calciumsalz der Dodecylbenzolsulfonsäure und Nonylphenylpolyoxyethylen

Die folgenden Beispiele erläutern die Herstellung der erfindungsgemäßen Verbindungen:

**Beispiel 1.01** (Verfahren E)

6-(6,6-Dimethyl-3,5,6,7-tetrahydropyrrolo[2,1-c][1,2,4]thiadiazol-3-ylidenimino)-4-(2-propinyl)-2H-1,4-benzoxazin-3(4H)-on

2,6 g 2-Amino-4,4-dimethylpyrrolinhydrochlorid wurden in 20 ml Methylendichlorid suspendiert. Hierzu wurde dann tropfenweise unter Rühren bei 0 °C eine Lösung von 0,75 g Natriumhydroxid in 5 ml Wasser zugetropft und 30 Minuten nachgerührt, wobei das Reaktionsgemisch klar wurde. Nun wurde eine Lösung von 6-Isothiocyanato-4-(2-propinyl)-2H-1,4-benzoxazin-3(4H)-on in 100 ml Methylendichlorid so zugetropft, daß 5 °C nicht überschritten wurde. Anschließend wurde 3 Stunden nachgerührt, wobei die Temperatur auf 20 °C anstieg. Unter Kühlung auf -20 °C wurde nun eine Lösung von 2,3 g Brom in 10 ml Methylenchlorid langsam zugetropft und 1 Stunde nachgerührt, wobei die Temperatur auf 10 °C anstieg. Danach wurde die Lösung mit 30 ml Wasser, 30 ml 5 %iger Natriumhydroxid-Lösung und mit 30 ml Wasser in der angegebenen Reihenfolge gewaschen. Die Methylendichloridphase wurde über wasserfreiem Magnesiumsulfat getrocknet, anschließend filtriert und eingeengt. Der Rückstand wurde auf einer Silicagelsäule chromatographisch gereinigt.

Ausbeute: 1,5 g = 25 % der Theorie
Fp.: 65 °C

**Herstellung des Ausgangsmaterials für Beispiel 1.01**

6-Isothiocyanato-4-(2-propinyl)-2H-1,4-benzoxazin-3(4H)-on

4,0 g 6-Amino-4-(2-propinyl)-2H-1,4-benzoxazin-3(4H)-on wurden in 100 ml Methylenchlorid gelöst und bei 5 °C mit einer Suspension von 2,60 g Calciumcarbonat in 20 ml Wasser versetzt. Anschließend wurden 2,9 g Thiophosgen so zugetropft, daß 5 °C Innentemperatur nicht überschritten wurden. Nach 10 Stunden bei Raumtemperatur wurden die Phasen getrennt. Die organische Phase wurde mit 50 ml Wasser gewaschen, über $MgSO_4$ getrocknet und das Lösungsmittel im Wasserstrahlvakuum abgezogen.

Ausbeute: 4,1 g = 84 % der Theorie
Fp.: 120-122 °C

In analoger Weise wurden die folgenden Verbindungen nach Verfahren E hergestellt:

| Beispiel Nr. | Name | Physikalische Konstante |
|---|---|---|
| 1.02 | 6-(6,6-Dimethyl-3,5,6,7-tetrahydropyrrolo-[2,1-c][1,2,4]thiadiazol-3-ylidenimino)-4-methyl-2H-1,4-benzoxazin-3(4H)-on | Fp.: 133°C |
| 1.03 | 6-(6,6-Dimethyl-3,5,6,7-tetrahydropyrrolo-[2,1-c][1,2,4]thiadiazol-3-ylidenimino)-7-fluor-4-(2-propinyl)-2H-1,4-benzoxazin-3(4H)-on | Fp.: 62°C |
| 1.04 | 4-(2,3-Dibrom-2-propenyl)-6-(6,6-dimethyl-3,5,6,7-tetrahydropyrrolo[2,1-c][1,2,4]thia-diazol-3-ylidenimino)-7-fluor-2H-1,4-benzoxazin-3(4H)-on | Fp.: 65°C |
| 1.05 | 5-(6,6-Dimethyl-5,6-dihydro-3H-thiazolo[2,3-c]-[1,2,4]thiadiazol-3-ylidenimino)-6-fluor-3-(2-propinyl)-2(3H)-benzthiazolon | Fp.: 195°C |
| 1.06 | 5-(6,6-Dimethyl-3,5,6,7-tetrahydropyrrolo[2,1-c]-[1,2,4]thiadiazol-3-ylidenimino)-6-fluor-3-(2-propinyl)-2(3H)-benzthiazolon | Fp.: 190°C |

| Beispiel Nr. | Name | Physikalische Konstante |
|---|---|---|
| 1.07 | 6-(6,6-Dimethyl-5,6-dihydro-3H-thiazolo-[2,3-c][1,2,4]thiadiazol-3-ylidenimino)-4-(2-propenyl)-2H-1,4-benzoxazin-3(4H)-on | Fp.: 124-26°C |
| 1.08 | 6-(6,6-Dimethyl-3,5,6,7-tetrahydropyrrolo[2,1-c]-[1,2,4]thiadiazol-3-ylidenimino)-4-(2-propenyl)-2H-1,4-benzoxazin-3(4H)-on | Fp.: 110°C |
| 1.09 | 6-(6,6-Dimethyl-5,6-dihydro-3H-thiazolo-[2,3-c][1,2,4]thiadiazol-3-ylidenimino)-4-(2-propinyl)-2H-1,4-benzoxazin-3(4H)-on | Fp.: 93°C |
| 1.10 | 6-(6,6-Dimethyl-5,6-dihydro-3H-thiazolo-[2,3-c][1,2,4]thiadiazol-3-ylidenimino)-4-propyl-2H-1,4-benzoxazin-3(4H)-on | Fp.: 53°C |
| 1.11 | 6-(6,6-Dimethyl-3,5,6,7-tetrahydropyrrolo-[2,1-c][1,2,4]thiadiazol-3-ylidenimino)-4-propyl-2H-1,4-benzoxazin-3(4H)-on | $n_D^{41}$: 1,6068 |
| 1.12 | 7-(6,6-Dimethyl-3,5,6,7-tetrahydropyrrolo-[2,1-c][1,2,4]thiadiazol-3-ylidenimino)-1-(2-propinyl)-2(1H)-chinoxalinon | Fp.: 168°C |
| 1.13 | 7-(6,6-Dimethyl-5,6-dihydro-3H-thiazolo-[2,3-c][1,2,4]thiadiazol-3-ylidenimino)-1-(2-propinyl)-2(1H)-chinoxalinon | Fp.: 174°C |
| 1.14 | 6-(6,6-Dimethyl-3,5,6,7-tetrahydropyrrolo-[2,1-c][1,2,4]thiadiazol-3-ylidenimino)-4-(2-propinyl)-2H-1,4-benzthiazin-3(4H)-on | Fp.: 65°C |

| Beispiel Nr. | Name | Physikalische Konstante |
|---|---|---|
| 1.15 | 6-(6,6-Dimethyl-5,6-dihydro-3H-thiazolo-[2,3-c][1,2,4]thiadiazol-3-ylidenimino)-7-fluor-4-(2-propinyl)-2H-1,4-benzoxazin-3(4H)-on | Fp.: 135-140°C |
| 1.16 | 6-(6,6-Dimethyl-3,5,6,7-tetrahydropyrrolo[2,1-c]-[1,2,4]thiadiazol-3-ylidenimino)-7-fluor-4-propyl-2H-1,4-benzoxazin-3(4H)-on | Fp.: 161°C |
| 1.17 | 6-(6,6-Dimethyl-5,6-dihydro-3H-thiazolo-[2,3-c][1,2,4]thiadiazol-3-ylidenimino)-7-fluor-4-propyl-2H-1,4-benzoxazin-3(4H)-on | Fp.: 156°C |
| 1.18 | 4-Cyanomethyl-6-(6,6-dimethyl-3,5,6,7-tetrahydropyrrolo[2,1-c][1,2,4]thiadiazol-3-ylidenimino)-2H-1,4-benzoxazin-3-(4H)-on | Fp.: 170-171°C |
| 1.19 | 6-(6-Methyl-3,5,6,7-tetrahydropyrrolo[2,1-c]-[1,2,4]thiadiazol-3-ylidenimino)-4-(2-propinyl)-2H-1,4-benzoxazin-3(4H)-on | Fp.: 171-172°C |
| 1.20 | 7-Fluor-6-(6-methyl-3,5,6,7-tetrahydropyrrolo-[2,1-c][1,2,4]thiadiazol-3-ylidenimino)-4-(2-propinyl)-2H-1,4-benzoxazin-3(4H)-on | Fp.: 161-162°C |
| 1.21 | 5-(6,6-Dimethyl-3,5,6,7-tetrahydropyrrolo[2,1-c]-[1,2,4]thiadiazol-3-ylidenimino)-3-propyl-2(3H)-benzoxazolon | Fp.: 113°C |
| 1.22 | 5-(6,6-Dimethyl-5,6-dihydro-3H-thiazolo[2,3-c]-[1,2,4]thiadiazol-3-ylidenimino)-3-propyl-2(3H)-benzoxazolon | Fp.: 113°C |

| Beispiel Nr. | Name | Physikalische Konstante |
|---|---|---|
| 1.23 | 5-(6,6-Dimethyl-3,5,6,7-tetrahydropyrrolo[2,1-c]-[1,2,4]thiadiazol-3-ylidenimino)-6-fluor-3-propyl-2(3H)-benzoxazolon | Fp.: 124-127°C |
| 1.24 | 5-(6,6-Dimethyl-5,6-dihydro-3H-thiazolo-[2,3-c][1,2,4]thiadiazol-3-ylidenimino)-6-fluor-3-propyl-2(3H)-benzoxazolon | Fp.: 141-142°C |
| 1.25 | 5-(6,6-Dimethyl-3,5,6,7-tetrahydropyrrolo[2,1-c]-[1,2,4]thiadiazol-3-ylidenimino)-3-(2-propinyl)-2(3H)-benzoxazolon | Fp.: 158°C |
| 1.26 | 5-(6,6-Dimethyl-5,6-dihydro-3H-thiazolo-[2,3-c][1,2,4]thiadiazol-3-ylidenimino)-3-(2-propinyl)-2(3H)-benzoxazolon | Fp.: 137°C |
| 1.27 | 5-(6,6-Dimethyl-3,5,6,7-tetrahydropyrrolo[2,1-c]-[1,2,4]thiadiazol-3-ylidenimino)-6-fluor-3-(2-propinyl)-2(3H)-benzoxazolon | Fp.: 169-173°C |
| 1.28 | 5-(6,6-Dimethyl-5,6-dihydro-3H-thiazolo-[2,3-c][1,2,4]thiadiazol-3-ylidenimino)-6-fluor-3-(2-propinyl)-2(3H)-benzoxazolon | Fp.: 138-142°C |
| 1.29 | 6-(6,6-Dimethyl-3,5,6,7-tetrahydropyrrolo[2,1-c]-[1,2,4]thiadiazol-3-ylidenimino)-3-methyl-1-propyl-1,3-dihydro-2(2H)-indolon | |
| 1.30 | 6-(6,6-Dimethyl-5,6-dihydro-3H-thiazolo-[2,3-c][1,2,4]thiadiazol-3-ylidenimino)-3-methyl-1-propyl-1,3-dihydro-2(2H)-indolon | |
| 1.31 | 7-(6,6-Dimethyl-3,5,6,7-tetrahydropyrrolo[2,1-c]-[1,2,4]thiadiazol-3-ylidenimino)-1-(2-propinyl)-3,4-dihydro-2(1H)-chinoxalinon | |

**Beispiel 2.01** (Verfahren A)

1-[6-Fluor-2-oxo-3-(2-propinyl)-2,3-dihydrobenzoxazol-5-yl]-3,4-dimethyl-3-pyrrolin-2,5(1H)-dion

Man legt 4 g 5-Amino-6-fluor-3-(2-propinyl)-2(3H)-benzoxazolon in 50 ml Eisessig vor und gibt 2,8 g 2,3-Dimethylmaleinsäureanhydrid hinzu. Es wird 8 Stunden bei 80 °C gerührt. Das abgekühlte Reaktionsgemisch wird auf 500 ml Eiswasser gegossen und die ausgefallenen Kristalle werden abgesaugt und mit Eiswasser nachgewaschen. Es wird im Vakuum bei 50 °C getrocknet.

Ausbeute: 2,3 g = 38 % der Theorie
Fp.: 165 °C

In analoger Weise wurden die folgenden Verbindungen nach Verfahren A hergestellt:

| Beispiel Nr. | Name | Physikalische Konstante |
|---|---|---|
| 2.02 | 1-[6-Fluor-2-oxo-3-(2-propinyl)-2,3-dihydrobenzthiazol-5-yl]-3,4-dimethyl-3-pyrrolin-2,5(1H)-dion | Fp.: 229 °C |
| 2.03 | 1-[3-Oxo-4-(2-propinyl)-3,4-dihydro-2H-1,4-benzthiazin-6-yl]-3,4-dimethyl-3-pyrrolin-2,5(1H)-dion | Fp.: 279 °C |
| 2.04 | 1-[2-Oxo-2,3-dihydro-1H-benzimidazol-5-yl]-3,4-dimethyl-3-pyrrolin-2,5(1H)-dion | Fp.: 280 °C |
| 2.05 | 1-[2-Oxo-1-(2-propinyl)-1H-chinoxalin-7-yl]-3,4-dimethyl-3-pyrrolin-2,5(1H)-dion | Fp.: 265 °C |
| 2.06 | 1-[2-Oxo-1-(2-propinyl)-3,4-dihydro-1H-chinoxalin-7-yl]-3,4-dimethyl-3-pyrrolin-2,5(1H)-dion | |

| Beispiel Nr. | Name | Physikalische Konstante |
|---|---|---|
| 2.07 | 1-[2-Oxo-3-(2-propinyl)-2,3-dihydrobenzoxazol-5-yl]-3,4-dimethyl-3-pyrrolin-2,5(1H)-dion | Fp.: 189°C |
| 2.08 | 1-(2-Oxo-3-propyl-2,3-dihydrobenzoxazol-5-yl)-3,4-dimethyl-3-pyrrolin-2,5(1H)-dion | Fp.: 110°C |
| 2.09 | 1-(3-Methyl-2-oxo-1-propyl-1,3-dihydro-2H-indol-6-yl)-3,4-dimethyl-3-pyrrolin-2,5(1H)-dion | Fp.: 180°C |
| 2.10 | 1-[3-Methyl-2-oxo-1-(1-methylethyl)-1,3-dihydro-2H-indol-6-yl]-3,4-dimethyl-3-pyrrolin-2,5(1H)-dion | Fp.: 142-144°C |

**Beispiel 3.01** (Verfahren D)

2-[7-Fluor-3-oxo-4-(2-propinyl)-3,4-dihydro-2H-1,4-benzoxazin-6-yl]-perhydroimidazo[1,5-a]pyridin-1,3-dion

Man löst 6,4 g 7-Fluor-4-(2-propinyl)-1,4-benzoxazin-3(4H)-on-6-yl-isocyanat in 50 ml Tetrahydrofuran und gibt 4,1 ml Piperidin-2-carbonsäureethylester hinzu. Es wird 10 Stunden am Rückfluß gekocht. Nach dem Abkühlen wird das Lösungsmittel im Vakuum abgezogen und der Rückstand über eine Kieselgelsäule mit Eissigester/Hexan getrennt.

Ausbeute: 4,8 g = 52 % der Theorie
Fp.: 202-204 °C

In analoger Weise wurden die folgenden Verbindungen nach Verfahren D hergestellt:

| Beispiel Nr. | Name | Physikalische Konstante |
|---|---|---|
| 3.02 | 2-[3-Oxo-4-(2-propinyl)-3,4-dihydro-2H-1,4-benzoxazin-6-yl]-perhydroimidazo[1,5-a]-pyridin-1,3-dion | Fp.: 180-185°C |
| 3.03 | 2-[3-Oxo-4-(2-propenyl)-3,4-dihydro-2H-1,4-benzoxazin-6-yl]-perhydroimidazo[1,5-a]-pyridin-1,3-dion | Fp.: 157-158°C |
| 3.04 | 2-[7-Fluor-3-oxo-4-(2-propinyl)-3,4-dihydro-2H-1,4-benzoxazin-6-yl]-3-thioxoperhydro-imidazo[1,5-a]pyridin-1-on | Fp.: 151°C |
| 3.05 | 2-[6-Fluor-2-oxo-3-(2-propinyl)-2,3-dihydro-benzoxazol-5-yl]-3-thioxoperhydroimidazo-[1,5-a]pyridin-1-on | Fp.: 189-195°C |

| Beispiel Nr. | Name | Physikalische Konstante |
|---|---|---|
| 3.06 | 2-(7-Fluor-3-oxo-4-propyl-3,4-dihydro-2H-1,4-benzoxazin-6-yl)-perhydroimidazo[1,5-a]-pyridin-1,3-dion | |
| 3.07 | 2-(2-Oxo-1-(2-propinyl)-1H-chinoxalin-7-yl]-perhydroimidazo[1,5-a]pyridin-1,3-dion | Fp.: 199-200 °C |
| 3.08 | 2-[2-Oxo-1-(2-propinyl)-3,4-dihydro-1H-chinoxalin-7-yl]-perhydroimidazo[1,5-a]pyridin-1,3-dion | |
| 3.09 | 2-[2-Oxo-3-(2-propinyl)-2,3-dihydro-benzoxazol-5-yl]-perhydroimidazo[1,5-a]pyridin-1,3-dion | Fp.: 179 °C |
| 3.10 | 2-(6-Fluor-2-oxo-3-(2-propinyl)-2,3-dihydro-benzoxazol-5-yl]-perhydroimidazo[1,5-a]pyridin-1,3-dion | |
| 3.11 | 2-(3-Methyl-2-oxo-1-propyl-1,3-dihydro-2H-indol-6-yl)-perhydroimidazo[1,5-a]pyridin-1,3-dion | |

**Beispiel 4.01** (Verfahren K)

3-(3-Oxo-4-propyl-3,4-dihydro-2H-1,4-benzoxazin-6-yl)-5,6,7,8-tetrahydrochinazolin-2,4(1H,3H)-dion

2,0 g Natriummetall werden in 100 ml Ethanol gelöst, 4,4 g 2-[3-(3-Oxo-4-propyl-3,4-dihydro-(2H)-1,4-benzoxazin-6-yl)-ureido]-1-cyclohexencarbonsäureethylester, gelöst in 50 ml Ethanol, zugegeben und die Reaktionsmischung 1 Stunde zum Sieden erhitzt. Nach Beendigung der Reaktion wird die Lösung eingeengt und der Rückstand in 300 ml Wasser aufgenommen. Man filtriert den Niederschlag ab und säuert das Filtrat mit Salzsäure an. Das ausgefallene Produkt wird abgesaugt, mit Wasser gewaschen und aus Essigester/Diisopropylether umkristallisiert.

Ausbeute: 3,56 g = 56 % der Theorie
Fp.: > 250 °C

**Beispiel 4.02** (Verfahren K)

1-Methyl-3-(4-propyl-3,4-dihydro-2H-1,4-benzoxazin-6-yl)-5,6,7,8-tetrahydrochinazolin-2,4(1H,3H)-dion

2,0 g 3-(4-Propyl-1,4-benzoxazin-3(4H)-on-6-yl)-5,6,7,8-tetrahydrochinazolin-2,4(1H,3H)-dion werden in 20 ml Dimethylformamid gelöst, 0,17 g 80%ige Natriumhydrid-Suspension zugegeben und 1 Stunde bei 60 °C gerührt. Man läßt auf Raumtemperatur abkühlen und gibt 0,77 g Jodmethan hinzu. Es wird 2 Stunden auf 60 °C erhitzt. Nach dem Abkühlen werden die ausgefallenen Kristalle abgesaugt und aus Diisopropylether/Essigester umkristallisiert.

Ausbeute: 1,3 g = 65 % der Theorie
Fp.: 218 °C

**Beispiel 5.01** (Verfahren I)

2-[6-Fluor-2-oxo-3-(2-propinyl)-2,3-dihydrobenzthiazol-5-yl]-2,3,5,6,7,8-hexahydro-1,2,4-triazolo[4,3-a]pyridin-3-on

3,6 g 5-Hydrazino-6-fluor-3-(2-propinyl)-benzthiazolon werden in 40 ml Xylol gelöst, 2,8 g 1-Ethoxycarbonyl-2-piperidon und 1 g Phosphorpentoxid zugefügt und die Mischung 2 Stunden am Rückfluß gekocht. Nach dem Abkühlen wird das Reaktionsgemisch auf 100 ml Wasser gegeben und die organische Phase abgetrennt. Die organische Phase wird mit 40 ml Kaliumhydrogencarbonatlösung gewaschen, über Magnesiumsulat getrocknet und eingeengt. Der Rückstand wird über Kieselgel mit Essigester/Hexan-Gemischen als Elutionsmittel chromatographiert.

Ausbeute: 380 mg = 6,7 % der Theorie
Fp.: 154-160 °C

**Herstellung des Ausgangsmaterials für Beispiel 5.01**

5-Hydrazino-6-fluor-3-(2-propinyl)-benzthiazolon

Man löst 4 g 5-Amino-6-fluor-3-(2-propinyl)-2(3H)-benzthiazolon in 50 ml konzentrierter Salzsäure und rührt etwa 30 Minuten bei Raumtemperatur. Es wird auf -5 bis +5 °C herabgekühlt und tropfenweise eine Lösung von 1,3 g Natriumnitrit in 5,6 ml Wasser zugegeben. Es wird 30 Minuten nachgerührt und anschließend auf -30 °C gekühlt. Nun wird eine Lösung aus 6,8 g Zinn(II)-chlorid in 15 ml konzentrierter Salzsäure langsam zugetropft. Es wird 2 Stunden nachgerührt, wobei die Temperatur zwischen -10 und 0 °C gehalten wird. Man filtriert den Feststoff ab, neutralisiert und extrahiert dreimal mit 50 ml Essigsäureethylester. Es wird über Magnesiumsulfat getrocknet und das Lösungsmittel im Vakuum abgezogen.

Ausbeute: 2,6 g = 61 % der Theorie

In analoger Weise werden die folgenden Verbindungen nach Verfahren I hergestellt:

| Beispiel Nr. | Name | Physikalische Konstante |
|---|---|---|
| 5.02 | 2-[2-Oxo-3-(2-propinyl)-2,3-dihydrobenzoxazol-5-yl]-2,3,5,6,7,8-hexahydro-1,2,4-triazolo-[4,3-a]pyridin-3-on | Fp.: 190°C |
| 5.03 | 2-[7-Fluor-3-oxo-4-(2-propinyl)-3,4-dihydro-2H-1,4-benzoxazin-6-yl]-2,3,5,6,7,8-hexahydro-1,2,4-triazolo[4,3-a]pyridin-3-on | Fp.: 194-196°C |
| 5.04 | 2-[6-Fluor-2-oxo-1-(2-propinyl)-3,4-dihydro-1H-chinoxalin-7-yl]-2,3,5,6,7,8-hexahydro-1,2,4-triazolo[4,3-a]pyridin-3-on | |
| 5.05 | 2-[6-Fluor-2-oxo-1-(2-propinyl)-1H-chinoxalin-7-yl]-2,3,5,6,7,8-hexahydro-1,2,4-triazolo-[4,3-a]pyridin-3-on | Fp.: 210-211°C |
| 5.06 | 2-[6-Fluor-2-oxo-3,4-dihydro-1H-chinoxalin-7-yl]-2,3,5,6,7,8-hexahydro-1,2,4-triazolo[4,3-a]-pyridin-3-on | Fp.: 190-192°C |

**Beispiel 6.01** (Verfahren B)

2-[7-Fluor-3-oxo-4-(2-propinyl)-3,4-dihydro-2H-1,4-benzoxazol-6-yl]-4,5,6,7-tetrahydro-2H-1,2,3-triazolo[3,4-a]pyridin-8-ium-3-olat

5.0 g 6-Amino-7-fluor-4-(2-propinyl)-2H-1,4-benzoxazin-3(4H)-on werden in einem Gemisch aus 12,2 ml konzentrierter Salzsäure und 52 ml Wasser suspendiert und auf -10° bis -5°C herabgekühlt. Es wird eine Lösung von 1,74 g Natriumnitrit in 6 ml Wasser tropfenweise so zugegeben, daß die Temperatur der Reaktionsmischung -5°C nicht übersteigt. Nach beendeter Zugabe wird 1 Stunde bei -5°C nachgerührt und überschüssige salpetrige Säure mit Harnstoff zerstört, bis der Test mit Jodstärkepapier negativ ist. Die so erhaltene Lösung wird auf 0°C erwärmt und zu einer eisgekühlten Lösung von 2,9 g Pipecolinsäure und 10,5 ml Triethylamin in 38 ml Wasser getropft. Nach beendeter Zugabe wird 1 Stunde bei 0°C nachgerührt und die Reaktionsmischung mehrfach mit Methylenchlorid extrahiert. Die vereinigten organischen Phasen werden über Magnesiumsulfat getrocknet und das Lösungsmittel eingedampft. Der Rückstand wird in 60 ml Ether aufgenommen und mit 5,2 ml Essigsäureanhydrid und 2,6 ml Pyridin versetzt. Man läßt über Nacht bei Raumtemperatur rühren und gießt anschließend auf 150 ml Eiswasser. Es wird mit Essigester extrahiert, mit Magnesiumsulfat getrocknet und das Lösungsmittel abgedampft. Der Rückstand wird über Kieselgel mit dem Elutionsmittel Essigester/Methanol = 95 : 5 chromatographiert.

Ausbeute: 1,0 g = 13 % der Theorie
Fp.: 202-203°C

In analoger Weise wurden die folgenden Verbindungen nach Verfahren B hergestellt:

| Beispiel Nr. | Name | Physikalische Konstante |
|---|---|---|
| 6.02 | 2-[6-Fluor-2-oxo-3-(2-propinyl)-2,3-dihydro-benzoxazol-5-yl]-4,5,6,7-tetrahydro-2H-1,2,3-triazolo[3,4-a]pyridin-8-ium-3-olat | Fp.: 203 °C |
| 6.03 | 2-[6-Fluor-2-oxo-3-(2-propinyl)-2,3-dihydro-benzthiazol-5-yl]-4,5,6,7-tetrahydro-2H-1,2,3-triazolo[3,4-a]pyridin-8-ium-3-olat | Fp.: 210 °C |

Die folgenden Beispiele erläutern die Anwendungsmöglichkeiten der erfindungsgemäßen Verbindungen:

**Beispiel A**

Im Gewächshaus wurden die aufgeführten Pflanzenspezies nach dem Auflaufen mit den aufgeführten Verbindungen in einer Aufwandmenge von 0,1 kg Wirkstoff/ha behandelt. Die Verbindungen wurde zu diesem Zweck als Emulsionen oder Suspensionen mit 500 Litern Wasser/ha gleichmäßig über die Pflanzen versprüht. Hier zeigten drei Wochen nach der Behandlung die erfindungsgemäßen Verbindungen eine hohe Kulturpflanzenselektivität bei ausgezeichneter Wirkung gegen das Unkraut.

In der folgenden Tabelle bedeuten:

0 = nicht geschädigt
4 = total vernichtet
TRZAX = Triticum aestivum
BRSSS = Brassica sp.
SOLSS = Solanum sp.
HELAN = Helianthus annuus
ABUTH = Abutilon theophrasti
MATCH = Matricaria chamomilla
VIOSS = Viola sp.
IPOSS = Ipomoea purpurea
VERPE = Veronica persica
GALAP = Galium aparine

| Erfindungsgemäße Verbindungen | TRZAX | BRSSS | SOLSS | HELAN | ABUTH | MATCH | VIOSS | IPOSS | VERPE | GALAP |
|---|---|---|---|---|---|---|---|---|---|---|
| Beispiel 1.01 | 0 | 4 | 4 | 4 | 4 | 4 | 4 | 4 | 4 | 4 |
| Beispiel 1.02 | 0 | 3 | 4 | 4 | 4 | 4 | 4 | 4 | 4 | 4 |
| Beispiel 1.03 | 0 | 4 | 4 | 4 | 4 | 4 | 4 | 4 | 4 | 4 |
| Beispiel 1.04 | 0 | 4 | 4 | 4 | 4 | 2 | 2 | 4 | 4 | 4 |
| Beispiel 1.05 | 1 | 1 | 4 | 3 | 4 | 4 | 4 | 4 | 4 | 3 |
| Beispiel 1.06 | 0 | 1 | 4 | 3 | 4 | 4 | 3 | 4 | 3 | 2 |
| Beispiel 1.08 | 0 | 2 | - | 3 | 4 | 3 | 2 | 4 | 4 | - |
| Beispiel 1.09 | 0 | 3 | - | 4 | 4 | 4 | 4 | 4 | 4 | 3 |
| Beispiel 1.10 | 1 | 1 | - | 3 | 4 | 4 | 3 | 4 | 4 | 3 |
| Beispiel 1.11 | 0 | 3 | - | 3 | 4 | 4 | - | 4 | 4 | 3 |
| Beispiel 1.12 | 0 | 3 | 4 | - | 4 | 4 | - | 4 | 4 | 2 |
| Beispiel 1.15 | 1 | 3 | 4 | - | 4 | 4 | 4 | 4 | 3 | 3 |
| Beispiel 1.16 | 1 | 4 | 4 | - | 4 | 3 | 3 | 3 | 3 | 2 |
| Beispiel 1.17 | 0 | 2 | 4 | - | 4 | 3 | 3 | 3 | 2 | 2 |
| Beispiel 1.18 | 1 | 3 | 3 | - | 4 | 3 | 2 | 3 | 4 | 1 |
| Beispiel 1.19 | 1 | 3 | 4 | - | 4 | 3 | 3 | 3 | 4 | 2 |
| Beispiel 1.20 | 1 | 4 | 4 | - | 4 | 4 | 3 | 4 | 4 | 4 |
| Beispiel 1.23 | 0 | 3 | 4 | - | 4 | 3 | 2 | 3 | 3 | 3 |
| Beispiel 1.27 | 0 | 2 | 4 | - | 4 | 3 | 3 | 4 | 3 | 1 |
| Beispiel 1.28 | 0 | 1 | 4 | - | 4 | 3 | 3 | 4 | 2 | 2 |
| Unbehandelt | 0 | 0 | 0 | 0 | 0 | 0 | 0 | 0 | 0 | 0 |

**Beispiel B**

Im Gewächshaus wurden die in der Tabelle aufgeführten Verbindungen mit der ebenfalls erwähnten Aufwandmenge appliziert. Hierzu wurden die Wirkstoffe in Gefäße mit 1500 ml Wasser gegeben. Als Testpflanzenart wurde Echinochloa crus-galli (ECHCG) im 2- bis 5-Blatt-Stadium eingesetzt. Drei Wochen nach der Behandlung zeigte sich, daß die erfindungsgemäßen Verbindungen stark wirksam gegen Echinochloa crus-galli sind.

In der Tabelle bedeuten:

0 = keine Schädigung

1 = schwache Schädigung

2 = mittlere Schädigung
3 = starke Schädigung
4 = total vernichtet

| Erfindungsgemäße Verbindungen | Wasserapplikation ppm | ECHCG |
|---|---|---|
| Beispiel 1.01 | 3 | 3 |
| Beispiel 1.02 | 3 | 4 |
| Unbehandelt | | 0 |

**Beispiel C**

Im Gewächshaus wurden die aufgeführten Pflanzenspezies nach dem Auflaufen mit den aufgeführten Verbindungen in einer Aufwandmenge von 0,01 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigten drei Wochen nach der Behandlung die erfindungsgemäßen Verbindungen eine hohe Kulturpflanzenselektivität bei ausgezeichneter Wirkung gegen das Unkraut. Das Vergleichsmittel zeigte nicht die hohe Effektivität.

In der folgenden Tabelle bedeuten:
0 = nicht geschädigt
4 = total geschädigt
TRZAX = Triticum aestivum
ZEAMX = Zea mays
VIOSS = Viola sp.
VERPE = Veronica persica
SOLSS = Solanium sp.
SEBEX = Sesbania exaltata
IPOSS = Ipomoea purpurea
ABUTH = Abutilon theophrasti

| Erfindungsgemäße Verbindungen | TRZAX | ZEAMX | VIOSS | VERPE | SOLSS | SEBEX | IPOSS | ABUTH |
|---|---|---|---|---|---|---|---|---|
| Beispiel 1.01 | 0 | 0 | 3 | 3 | 4 | 4 | 4 | 4 |
| Beispiel 2.01 | 0 | 0 | 3 | 4 | 4 | 2 | 4 | 4 |
| Beispiel 2.02 | 1 | 0 | 3 | 3 | 4 | - | - | 4 |
| Beispiel 3.01 | - | 1 | 2 | 4 | 4 | 4 | 4 | 4 |
| Beispiel 3.02 | 0 | 0 | - | 3 | - | - | 3 | 4 |
| Beispiel 6.01 | 2 | 0 | 3 | 4 | 4 | 4 | 4 | 4 |
| Unbehandelt | 0 | 0 | 0 | 0 | 0 | 0 | 0 | 0 |
| **Vergleichsmittel** | | | | | | | | |
| Oxadiazon | 0 | 0 | 0 | 0 | 0 | 1 | 1 | 2 |

**Beispiel D**

Im Gewächshaus wurden die aufgeführten Pflanzenspezies vor dem Auflaufen mit den aufgeführten Verbindungen in einer Aufwandmenge von 0,1 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigten drei Wochen nach der Behandlung die erfindungsgemäßen Verbindungen eine hohe Kulturpflanzenselektivität in Zuckerrübe, Weizen und Mais bei ausgezeichneter Wirkung gegen das Unkraut. Das Vergleichsmittel zeigte nicht die gleichhohe Selektivität.

In der folgenden Tabelle bedeuten:
0 = keine Schädigung
1 = 1 - 24 % Schädigung
2 = 25 - 74 % Schädigung
3 = 75 - 89 % Schädigung
4 = 90 - 100 % Schädigung
BEAVX = Beta vulgaris altissima
TRZAX = Triticum aestivum
ZEAMX = Zea mays
GALAP = Galium aparine
MATCH = Matricaria chamomilla

| Erfindungsgemäße Verbindungen | BEAVX | TRZAX | ZEAMX | GALAP | MATCH |
|---|---|---|---|---|---|
| Beispiel 3.04 | 0 | - | - | - | 4 |
| Beispiel 4.01 | - | 0 | 0 | 4 | - |
| Unbehandelt | 0 | 0 | 0 | 0 | 0 |
| **Vergleichsmittel** | | | | | |
| Oxadiazon | 1 | 1 | 1 | 0 | 4 |

**Beispiel E**

Im Gewächshaus wurden die aufgeführten Pflanzenspezies nach dem Auflaufen mit den aufgeführten Verbindunen in einer Aufwandmenge von 0,1 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigten zwei Wochen nach der Behandlung die erfindungsgemäßen Verbindungen eine hohe Kulturpflanzenselektivität in Mais bei ausgezeichneter Wirkung gegen das Unkraut. Das Vergleichsmittel zeigte nicht die gleichhohe Selektivität.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
ZEAMX = Zea mays
ABUTH = Abutilon theophrasti
MATCH = Matricaria chamomilla
POLSS = Polygonum sp.
SOLSS = Solanum ssp.
VERPE = Veronica persica

| Erfindungsgemäße Verbindungen | ZEAMX | ABUTH | MATCH | POLSS | SOLSS | VERPE |
|---|---|---|---|---|---|---|
| Beispiel 3.04 | 0 | - | 3 | 3 | 4 | 3 |
| Beispiel 3.05 | 0 | 4 | - | 4 | 3 | 4 |
| Unbehandelt | 0 | 0 | 0 | 0 | 0 | 0 |
| **Vergleichsmittel** | | | | | | |
| Oxadiazon | 1 | 0 | 4 | 3 | 4 | 4 |

**Beispiel F**

Im Gewächshaus wurden die aufgeführten Pflanzenspezies vor dem Auflaufen mit den aufgeführten Verbindungen in einer Aufwandmenge von 1,0 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigten eine Woche nach der Behandlung die erfindungsgemäßen Verbindungen eine ausgezeichnete Wirkung gegen das Unkraut.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
ALOMY = Alopecurus myosuroides
AVEFA = Avena fatua
SETVI = Setaria viridis
CYPES = Cyperus esculentus
ABUTH = Abutilon theophrasti
IPOSS = Ipomoea purpurea
MATCH = Matricaria chamomilla

| Erfindungsgemäße Verbindungen | ALOMY | AVEFA | SETVI | CYPES | ABUTH | IPOSS | MATCH |
|---|---|---|---|---|---|---|---|
| Beispiel 6.01 | 4 | 4 | 4 | 4 | 4 | 4 | 4 |
| Beispiel 6.02 | 4 | 4 | 4 | 3 | 4 | 4 | 4 |
| Beispiel 6.03 | 4 | 4 | 4 | 4 | 4 | 4 | 4 |
| Unbehandelt | 0 | 0 | 0 | 0 | 0 | 0 | 0 |

**Beispiel G**

Im Gewächshaus wurden die aufgeführten Pflanzenspezies nach dem Auflaufen mit den aufgeführten Verbindungen in einer Aufwandmenge von 0,1 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigten eine Woche nach der Behandlung die erfindungsgemäßen Verbindungen eine hohe Kulturpflanzenselektivität in Weizen bei ausgezeichneter Wirkung gegen das Unkraut.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
TRZAX = Triticum aestivum
ABUTH = Abutilon theophrasti
GALAP = Galium aparine
IPOSS = Ipomoea purpurea
MATCH = Matricaria chamomilla

| Erfindungsgemäße Verbindungen | TRZAX | ABUTH | GALAP | IPOSS | MATCH |
|---|---|---|---|---|---|
| Beispiel 6.01 | 1 | 4 | 3 | 4 | 4 |
| Beispiel 6.02 | 1 | 4 | 3 | 3 | 3 |
| Unbehandelt | 0 | 0 | 0 | 0 | 0 |

**Beispiel H**

Im Gewächshaus wurden die aufgeführten Pflanzenspezies nach dem Auflaufen mit den aufgeführten Verbindungen in einer Aufwandmenge von 0,3 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigten zwei Wochen nach der Behandlung die erfindungsgemäßen Verbindungen eine hohe Kulturpflanzenselektivität in Weizen und Mais bei ausgezeichneter Wirkung gegen das Unkraut.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
TRZAX = Triticum aestivum
ZEAMX = Zea mays
ABUTH = Abutilon theophrasti
IPOSS = Ipomoea purpurea
MATCH = Matricaria chamomilla
SOLSS = Solanum sp.
VIOSS = Viola sp.

| Erfindungsgemäße Verbindungen | TRZAX | ZEAMX | ABUTH | IPOSS | MATCH | SOLSS | VIOSS |
|---|---|---|---|---|---|---|---|
| Beispiel 1.13 | 0 | 0 | 3 | 3 | 3 | 4 | 2 |
| Beispiel 1.14 | 0 | 0 | 4 | 4 | 2 | 4 | 1 |
| Beispiel 1.21 | 0 | 0 | 4 | 3 | 3 | 4 | 2 |
| Beispiel 1.22 | 0 | 0 | 4 | 3 | 3 | 4 | 3 |
| Beispiel 1.24 | 0 | 0 | 4 | 4 | 3 | 4 | 3 |
| Beispiel 1.25 | 0 | 0 | 4 | 4 | 3 | 4 | 3 |
| Beispiel 1.26 | 0 | 0 | 3 | 3 | 3 | 4 | 3 |
| Beispiel 2.05 | 2 | 1 | 4 | 4 | 3 | 4 | 1 |
| Unbehandelt | 0 | 0 | 0 | 0 | 0 | 0 | 0 |

## Patentansprüche

**Patentansprüche für folgende Vertragsstaaten : AT, BE, CH, DE, FR, GB, GR, IT, LI, LU, NL, SE**

**1.** Heterocyclisch substituierte Azole und Azine der allgemeinen Formel I

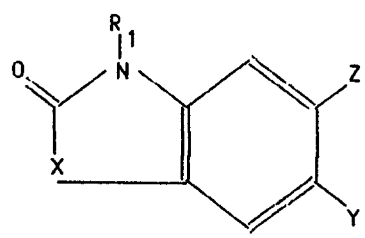

(I),

in der

$R_1$ ein Wasserstoffatom, eine $C_1$-$C_5$-Alkylgruppe, eine $C_3$-$C_5$-Alkenylgruppe, eine $C_3$-$C_5$-Alkinylgruppe, eine Halogen-$C_1$-$C_4$-alkylgruppe, eine Halogen-$C_3$-$C_5$-alkenylgruppe, eine Halogen-$C_3$-$C_5$-alkinylgruppe oder eine Cyano-$C_1$-$C_3$-alkylgruppe,

Y ein Wasserstoff- oder Fluoratom,

X eine der Gruppen $(CR_2R_3)_n$-W oder $(CR_2)=V$, wobei V beziehungsweise W direkt am Phenylkern gebunden sind,

n 0 oder 1,

W eine der Gruppen $CR_4R_5$ oder $NR_6$ oder ein Sauerstoff- oder Schwefelatom, mit der Ein-

schränkung, daß, wenn Z = $Z_1$ und n = 1 bedeuten, W nicht Sauerstoff ist,

V eine Gruppe $CR_4$ oder ein Stickstoffatom,

$R_2$, $R_3$, $R_4$ und $R_5$ gleich oder verschieden jeweils ein Wasserstoff- oder Halogenatom oder eine $C_1$-$C_3$-Alkyl- oder Halogen-$C_1$-$C_3$-alkylgruppe,

$R_6$ ein Wasserstoffatom,

Z einen heterocyclischen Rest der Formeln $Z_1$ und $Z_3$ bis $Z_8$

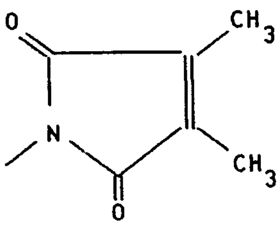

,

$Z_1$

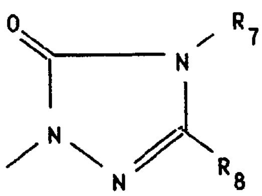

,

$Z_3$

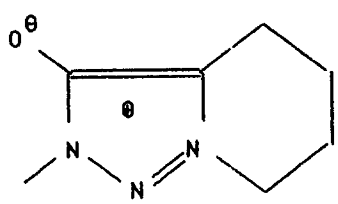

,

$Z_5$

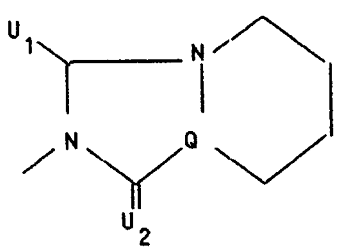

,

$Z_6$

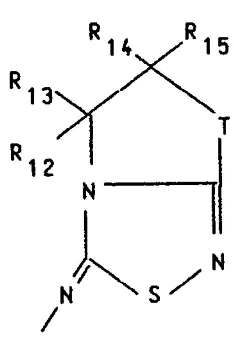

oder

$Z_7$

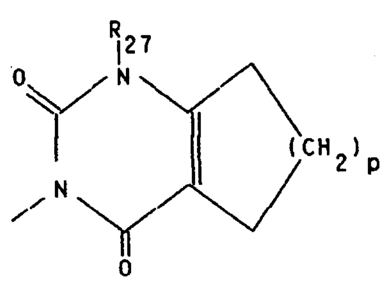

.

$Z_8$

Q die Gruppe CH, mit der Einschränkung, daß wenn n = 0 bedeutet, W nicht Schwefel ist,

$U_1$ ein Sauerstoff- oder Schwefelatom,

| | |
|---|---|
| $U_2$ | ein Sauerstoffatom, |
| $R_7$ und $R_8$ | gemeinsam mit den benachbarten Stickstoff- und Kohlenstoffatomen einen heterocyclischen 5- bis 7-gliedrigen Ring bildet, der gesättigt oder ungesättigt ist, |
| T | die Gruppe D-E, |
| D | eine Gruppe $(CR_{16}R_{17})_m$, |
| E | ein Schwefelatom oder die Gruppe $CR_{19}R_{20}$, |
| m | = 0, |
| p | = 2, |
| $R_{12}$, $R_{13}$, $R_{14}$, $R_{15}$, $R_{16}$, $R_{17}$, $R_{18}$, $R_{19}$ und $R_{20}$ | ein Wasserstoffatom oder eine $C_1$-$C_4$-Alkylgruppe und |
| $R_{27}$ | ein Wasserstoffatom oder eine $C_1$-$C_5$-Alkylgruppe bedeutet. |

**2.** Mittel mit herbizider Wirkung, gekennzeichnet durch einen Gehalt an mindestens einer Verbindung gemäß dem Anspruch 1.

**3.** Verwendung von Mitteln gemäß dem Anspruch 2 zur Bekämpfung monokotyler und dikotyler Unkrautarten in landwirtschaftlichen Hauptkulturen.

**Patentanspruch für folgenden Vertragsstaat : ES**

**1.** Verfahren zur Herstellung von heterocyclisch substituierten Azolen und Azinen der allgemeinen Formel I

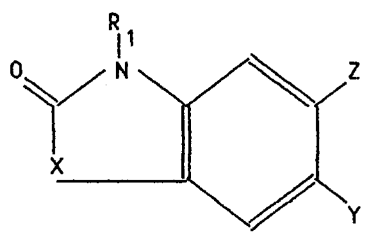

(I),

in der

| | |
|---|---|
| $R_1$ | ein Wasserstoffatom, eine $C_1$-$C_5$-Alkylgruppe, eine $C_3$-$C_5$-Alkenylgruppe, eine $C_3$-$C_5$-Alkinylgruppe, eine Halogen-$C_1$-$C_4$-alkylgruppe, eine Halogen-$C_3$-$C_5$-alkenylgruppe, eine Halogen-$C_3$-$C_5$-alkinylgruppe oder eine Cyano-$C_1$-$C_3$-alkylgruppe, |
| Y | ein Wasserstoff- oder Fluoratom, |
| X | eine der Gruppen $(CR_2R_3)_n$-W oder $(CR_2)=V$, wobei V beziehungsweise W direkt am Phenylkern gebunden sind, |
| n | 0 oder 1, |
| W | eine der Gruppen $CR_4R_5$ oder $NR_6$ oder ein Sauerstoff- oder Schwefelatom, mit der Einschränkung, daß, wenn Z = $Z_1$ und n = 1 bedeuten, W nicht Sauerstoff ist, |
| V | eine Gruppe $CR_4$ oder ein Stickstoffatom, |
| $R_2$, $R_3$, $R_4$ und $R_5$ | gleich oder verschieden jeweils ein Wasserstoff- oder Halogenatom oder eine $C_1$-$C_3$-Alkyl- oder Halogen-$C_1$-$C_3$-alkylgruppe, |

$R_6$ ein Wasserstoffatom,

Z einen heterocyclischen Rest der Formeln $Z_1$ und $Z_3$ bis $Z_8$

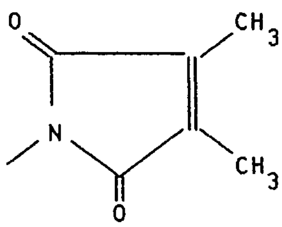

,

$Z_1$

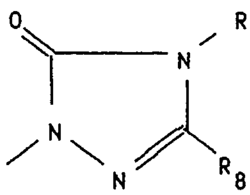

,

$Z_3$

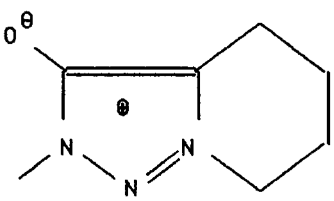

,

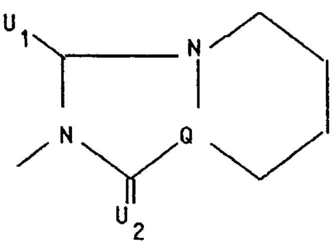

,

$Z_5$ $Z_6$

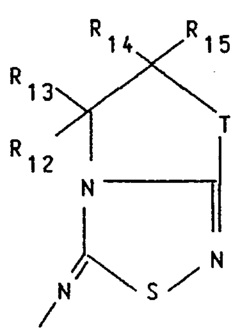

oder

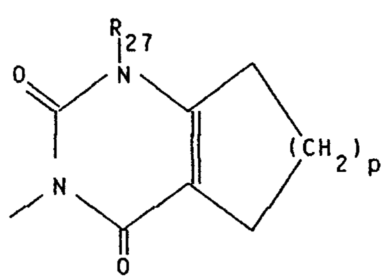

.

$Z_7$ $Z_8$

Q die Gruppe CH, mit der Einschränkung, daß wenn n = 0 bedeutet, W nicht Schwefel ist,

$U_1$ ein Sauerstoff- oder Schwefelatom,

$U_2$ ein Sauerstoffatom,

$R_7$ und $R_8$ gemeinsam mit den benachbarten Stickstoff- und Kohlenstoff atomen einen heterocyclischen 5- bis 7-gliedrigen Ring bildet, der gesättigt oder ungesättigt ist,

| | |
|---|---|
| T | die Gruppe D-E, |
| D | eine Gruppe $(CR_{16}R_{17})_m$, |
| E | ein Schwefelatom oder die Gruppe $CR_{19}R_{20}$, |
| m | = 0, |
| p | = 2, |
| $R_{12}$, $R_{13}$, $R_{14}$, $R_{15}$, $R_{16}$, $R_{17}$, $R_{18}$, $R_{19}$ und $R_{20}$ | ein Wasserstoffatom oder eine $C_1$-$C_4$-Alkylgruppe und |
| $R_{27}$ | ein Wasserstoffatom oder eine $C_1$-$C_5$-Alkylgruppe bedeutet, |

dadurch gekennzeichnet, daß man

A) falls Z einen 3,4-Dimethyl-3-pyrrolin-2,5-dion-1-yl-Rest darstellt, ein Anilin der allgemeinen Formel II

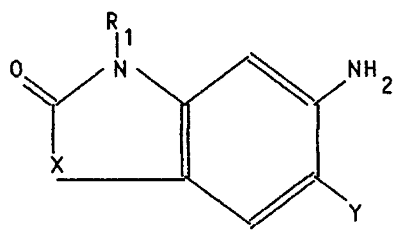

(II),

in der $R_1$, X und Y die unter der allgemeinen Formel I genannten Bedeutungen haben, mit 2,2'-Dimethylmaleinsäureanhydrid umsetzt,

B) falls Z einen 4,5,6,7-Tetrahydro-2H-1,2,3-triazolo[3,4-a]pyridin-8-ium-3-olat-2-yl-Rest darstellt, eine Verbindung der allgemeinen Formel II

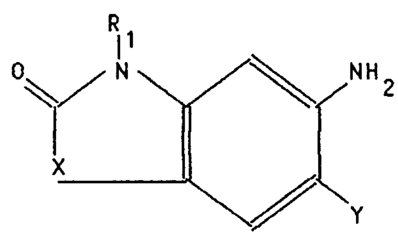

(II),

in der $R_1$, X und Y die unter der allgemeinen Formel I angegebenen Bedeutungen haben, mit salpetriger Säure diazotiert, mit Piperidin-2-carbonsäure umsetzt und mit Hilfe von dehydratisierenden Mitteln cyclisiert,

C) falls Z einen 2,3,5,6,7,8-Hexahydro-1H-imidazo[1,5-a]pyridin-1,3-dion-2-yl- oder einen 2,3,5,6,7,8-Hexahydro-1H-1,3,4-triazolo[1,2-a]pyridazin-1,3-dion-2-yl-Rest darstellt, eine Verbindung der allgemeinen Formel II

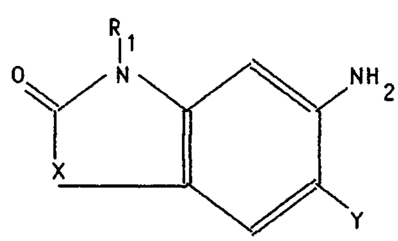

(II),

in der $R_1$, X und Y die unter der allgemeinen Formel I genannten Bedeutungen haben, mit einer Verbindung der allgemeinen Formel V oder VI

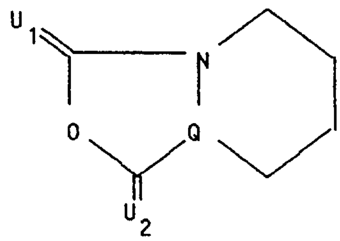

(V)

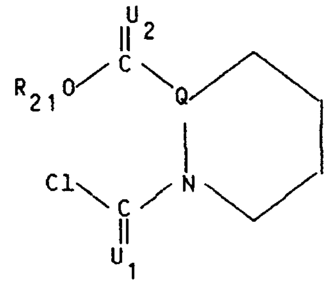

(VI),

in denen Q, $U_1$ und $U_2$ die unter der allgemeinen Formel I genannten Bedeutungen haben und $R_{21}$ einen $C_1$-$C_4$-Alkylrest darstellt, umsetzt,

D) falls Z einen 2,3,5,6,7,8-Hexahydro-1H-imidazo[1,5-a]pyridin-1,3-dion-2-yl- oder einen 2,3,5,6,7,8-Hexahydro-1H-1,3,4-triazolo[1,2-a]pyridazin-1,3-dion-2-yl-Rest darstellt, eine Verbindung der allgemeinen Formel III

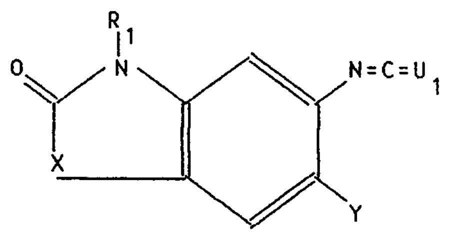

(III),

in der $R_1$, $U_1$, X und Y die unter der allgemeinen Formel I genannten Bedeutungen haben, mit einer Verbindung der allgemeinen Formel VII

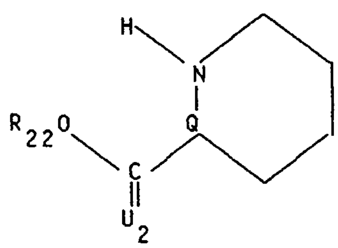

(VII)

in der Q und $U_2$ die unter der allgemeinen Formel I genannten Bedeutungen haben und $R_{22}$ einen $C_1$-$C_4$-Alkylrest darstellt, zur Reaktion bringt,

E) falls Z einen Rest der allgemeinen Formel

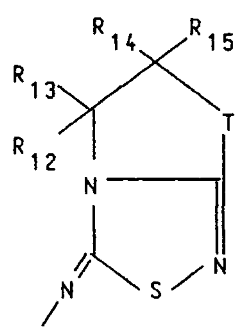

darstellt, in der $R_{12}$, $R_{13}$, $R_{14}$, $R_{15}$ und T die unter der allgemeinen Formel I genannten Bedeutungen haben, eine Verbindung der allgemeinen Formel IIIa

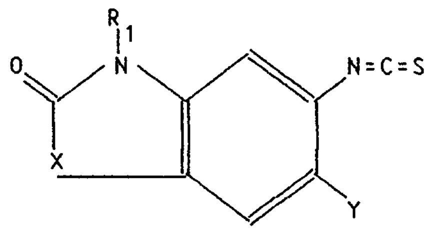

(IIIa),

in der $R_1$, X und Y die unter der allgemeinen Formel I genannten Bedeutungen haben, mit einer Verbindung der allgemeinen Formel VIII

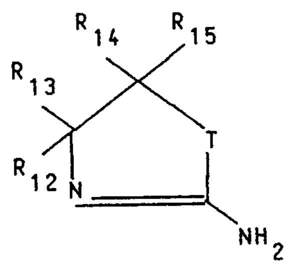

(VIII).

in der $R_{12}$, $R_{13}$, $R_{14}$, $R_{15}$ und T die unter der allgemeinen Formel I genannten Bedeutungen haben, umsetzt und in Gegenwart eines Oxidationsmittels zum Thiadiazolring cyclisiert,
I) falls Z einen 2H-1,2,4-Triazolin-3-on-2-yl-Rest darstellt, eine Verbindung der allgemeinen Formel IV

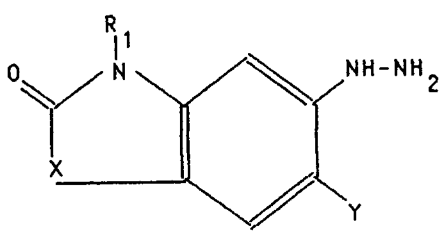

(IV).

in der $R_1$, X und Y die in der allgemeinen Formel I genannten Bedeutungen haben, mit einer Verbindung der allgemeinen Formel XI

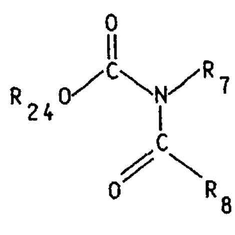

(XI),

worin $R_7$ und $R_8$ die unter der allgemeinen Formel I genannten Bedeutungen haben und und $R_{24}$ einen $C_1$-$C_4$-Alkylrest darstellt, umsetzt,
K) falls Z einen 5,6,7,8-Tetrahydrochinazolin-2,4(1H,3H)-dion-3-yl-Rest oder einen 1,2,4,5,6,7-Hexahydro-2,4-dioxo-3H-cyclopentapyrimidin-3-yl-Rest darstellt, eine Verbindung der allgemeinen Formel XXII

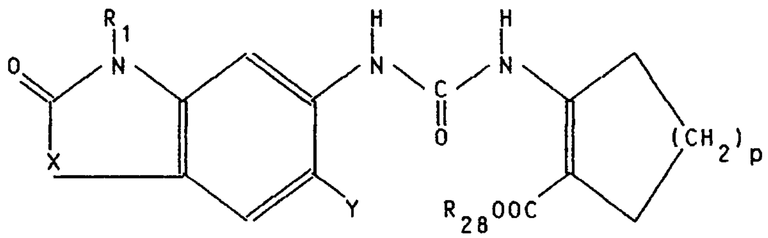

(XXII),

in der $R_1$, X, Y und p, die unter der allgemeinen Formel I angegebenen Bedeutungen haben und $R_{28}$ einen $C_1$-$C_4$-Alkylrest darstellt, unter basischen Bedingungen cyclisiert und gegebenenfalls mit einer Verbindung der allgemeinen Formel XXIII

$R_{27}$ - B (XXIII),

in der $R_{27}$ die unter der allgemeinen Formel I angegebene Bedeutung hat, aber nicht ein Wasserstoffatom darstellt, und B ein Halogenatom oder die p-Toluolsulfonyloxygruppe bedeutet, gegebenenfalls unter Verwendung eines säurebindenden Mittels umsetzt.

**Claims**

**Claims for the following Contracting States : AT, BE, CH, DE, FR, GB, GR, IT, LI, LU, NL, SE**

1. Heterocyclic substituted azoles and azine of general formula I

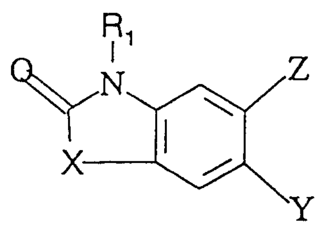

(I),

in which

| | |
|---|---|
| $R_1$ | is hydrogen, $C_{1-5}$-alkyl, $C_{3-5}$-alkenyl, $C_{3-5}$-alkynyl, halo-$C_{1-4}$-alkyl, halo-$C_{3-5}$-alkenyl, halo-$C_{3-5}$-alkynyl or cyano-$C_{1-3}$-alkyl, |
| Y | is hydrogen or fluorine, |
| X | is the group $(CR_2R_3)_n$-W or $(CR_2)=V$, in which V and W are bonded directly to the phenyl nucleus, |
| n | is 0 or 1, |
| W | is the group $CR_4R_5$ or $NR_6$ or oxygen or sulphur, with the proviso that when Z = $Z_1$ and n = 1, W is not oxygen, |
| V | is the group $CR_4$ or nitrogen, |
| $R_2$, $R_3$, $R_4$ and $R_5$ | are the same or different and are each hydrogen, halogen, $C_{1-3}$-alkyl or halo-$C_{1-3}$-alkyl, |
| $R_6$ | is hydrogen, |
| Z | is a heterocyclic group of formulae $Z_1$, $Z_3$ and $Z_5$ to $Z_8$ |

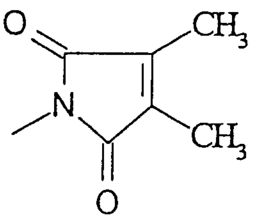

$Z_1$

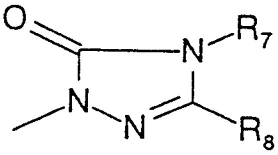

$Z_3$

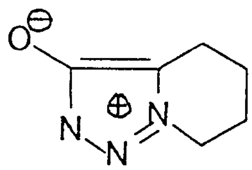

$Z_5$

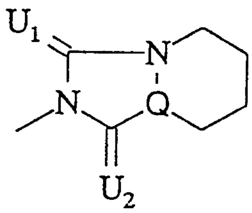

$Z_6$

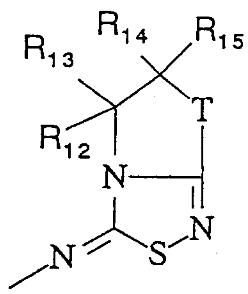

$Z_7$

or

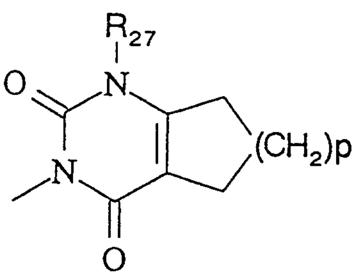

$Z_8$

| | |
|---|---|
| Q | is the group CH, with the proviso that when n = 0, W is not sulphur, |
| $U_1$ | is oxygen or sulphur, |
| $U_2$ | is oxygen, |
| $R_7$ and $R_8$ | together with the adjacent nitrogen and carbon atoms form a 5 to 7 membered heterocyclic ring, that is saturated or unsaturated, |
| T | is the group D-E, |
| D | is a group $(CR_{16}R_{17})_m$, |
| E | is sulphur or the group $CR_{19}R_{20}$, |
| m | is 0, |
| p | is 2, |
| $R_{12}$, $R_{13}$, $R_{14}$, $R_{15}$, $R_{16}$, $R_{17}$, $R_{18}$, $R_{19}$ and $R_{20}$ | are hydrogen or $C_{1-4}$-alkyl, and |
| $R_{27}$ | is hydrogen or $C_{1-5}$-alkyl. |

**2.** A herbicidal composition which comprises a compound according to claim 1, in admixture with carriers and diluents.

**3.** A method of combating weeds which comprises applying to the weeds or their locus a compound according to claim 1.

**Claim for the following Contracting State : ES**

**1.** Process for the preparation of heterocyclic substituted azoles and azines of general formula I

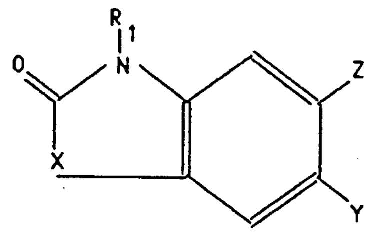

(I),

in which

| | |
|---|---|
| $R_1$ | is a hydrogen atom, $C_{1-5}$-alkyl, $C_{3-5}$-alkenyl, $C_{3-5}$-alkynyl, halo-$C_{1-4}$-alkyl, halo-$C_{3-5}$-alkenyl, halo-$C_{3-5}$-alkynyl or a cyano-$C_{1-3}$-alkyl group, |
| Y | is a hydrogen or fluorine atom, |
| X | is the group $(CR_2R_3)_n$-W or $(CR_2)=V$, in which V and W are bonded directly to the phenyl nucleus, |
| n | is 0 or 1, |
| W | is the group $CR_4R_5$ or $NR_6$ or is an oxygen or sulphur atom, with the proviso that when Z = $Z_1$ and n = 1, W is not oxygen, |
| V | is the group $CR_4$ or a nitrogen atom, |
| $R_2$, $R_3$, $R_4$ and $R_5$ | are the same or different and are each a hydrogen or a halogen atom or a $C_{1-3}$-alkyl or halo-$C_{1-3}$-alkyl group, |
| $R_6$ | is a hydrogen atom, |
| Z | is a heterocyclic group of formulas $Z_1$ and $Z_3$ to $Z_8$ |

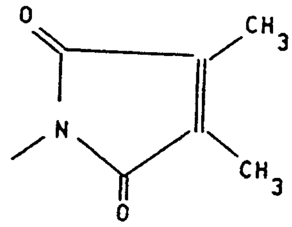

,

$Z_1$

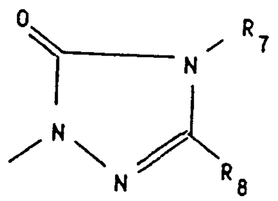

,

$Z_3$

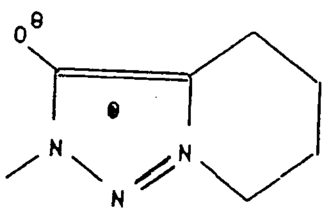

,

$Z_5$

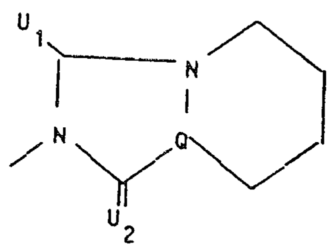

,

$Z_6$

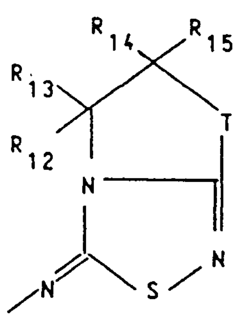

$Z_7$

or

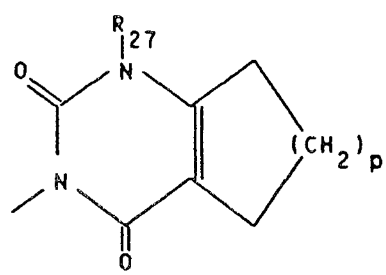

,

$Z_8$

| | |
|---|---|
| Q | is the group CH, with the proviso that when n = 0, W is not sulphur, |
| $U_1$ | is an oxygen or sulphur atom, |
| $U_2$ | is an oxygen atom, |
| $R_7$ and $R_8$ | together with the adjacent nitrogen and carbon atoms form a 5 to 7 membered heterocyclic ring, that is saturated or unsaturated, |
| T | is the group D-E, |
| D | is a group $(CR_{16}R_{17})_m$, |
| E | is a sulphur atom or the group $CR_{19}R_{20}$, |

| | |
|---|---|
| m | is 0, |
| p | is 2, |
| $R_{12}$, $R_{13}$, $R_{14}$, $R_{15}$, $R_{16}$, $R_{17}$, $R_{18}$, $R_{19}$ and $R_{20}$ | are a hydrogen atom or a $C_{1-4}$-alkyl group, and |
| $R_{27}$ | is a hydrogen atom or a $C_{1-5}$-alkyl group, characterized by |

A) when Z is a 3,4-dimethyl-3-pyrroline-2,5-dion-1-yl group, reacting an aniline of general formula II

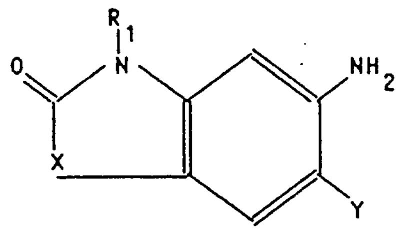

(II),

in which $R_1$, X and Y have the meanings given under general formula I, with 2,2'-dimethylmaleic anhydride,

B) when Z is a 4,5,6,7-tetrahydro-2H-1,2,3-triazolo[3,4-a]pyridin-8-ium-3-olat-2-yl group, diazotising a compound of general formula II

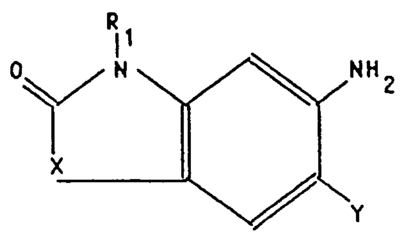

(II),

in which $R_1$, X and Y have the meanings given under general formula I, with nitrous acid, treating with piperidine-2-carboxylic acid, and cyclising by using a dehydrating agent,

C) when Z is a 2,3,5,6,7,8-hexahydro-1H-imidazo[1,5-a]pyridine-1,3-dion-2-yl or a 2,3,5,6,7,8-hexahydro-1H-1,3,4-triazolo[1,2-a]pyridazine-1,3-dion-2-yl-group, treating a compound of general formula II

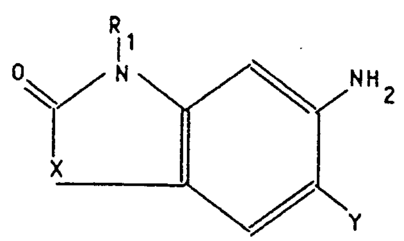

(II),

in which $R_1$, X and Y have the meanings given under general formula I, with a compound of general formula V or VI

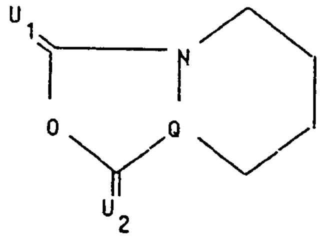 (V) 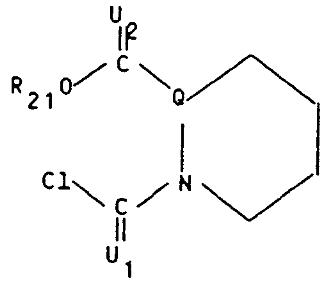 (VI),

in which Q, $U_1$ and $U_2$ have the meanings given under general formula I and $R_{21}$ is a $C_{1-4}$-alkyl group,

D) when Z is a 2,3,5,6,7,8-hexahydro-1H-imidazo[1,5-a]pyridine-1,3-dion-2-yl or a 2,3,5,6,7,8-hexahydro-1H-1,3,4-triazolo[1,2-a]pyridazine-1,3-dion-2-yl group, reacting a compound of general formula III

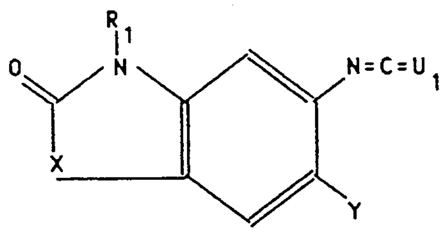 (III),

in which $R_1$, $U_1$, X and Y have the meanings given under general formula I, with a compound of general formula VII

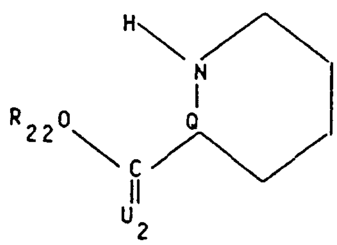 (VII),

in which Q and $U_2$ have the meanings given under general formula I and $R_{22}$ is a $C_{1-4}$-alkyl group,

E) when Z is a group of general formula

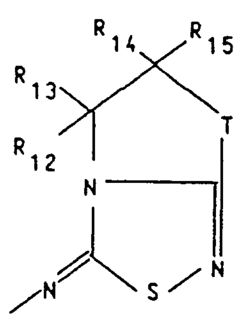 ,

in which $R_{12}$, $R_{13}$, $R_{14}$, $R_{15}$ and T have the meanings given under general formula I, reacting a compound of general formula IIIa

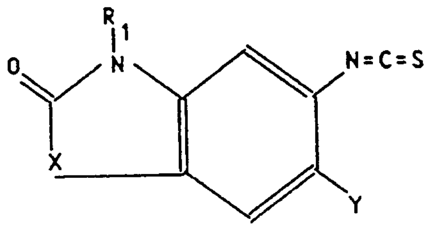

(III a),

in which $R_1$, X and Y have the meanings given under general formula I, with a compound of general formula VIII

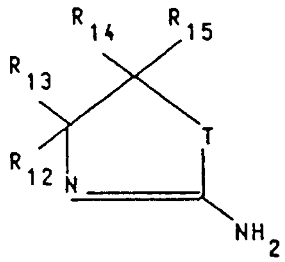

(VIII),

in which $R_{12}$, $R_{13}$, $R_{14}$, $R_{15}$ and T have the meanings given under general formal I, and then cyclising to a thiadiazole ring in the presence of an oxidising agent,
I) when Z is a 2H-1,2,4-triazolin-3-on-2-yl group, reacting a compound of general formula IV

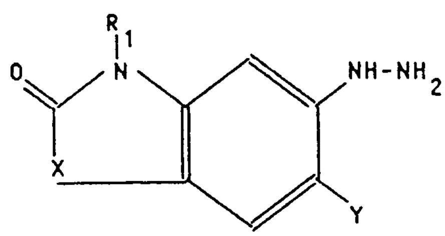

(IV),

in which $R_1$, X and Y have the meanings given under general formula I, with a compound of general formula XI

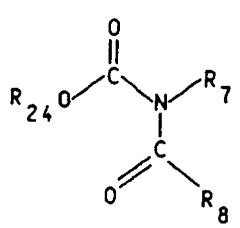

(XI) ,

in which $R_7$ and $R_8$ have the meanings given under general formula I and $R_{24}$ is a $C_{1-4}$-alkyl group,
K) when Z is a 5,6,7,8-tetrahydroquinazoline-2,4(1H,3H)-dion-3-yl group or a 1,2,4,5,6,7-hexahydro-2,4-dioxo-3H-cyclopentapyrimidin-3-yl group, cyclising a compound of general formula XXII

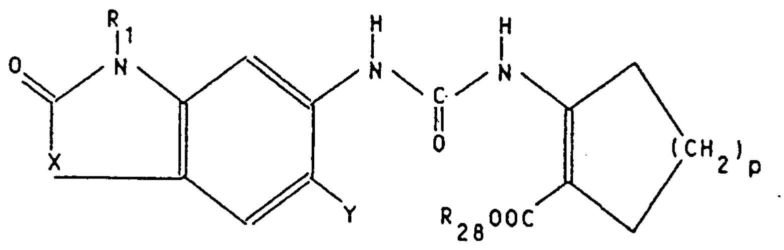

(XXII),

in which $R_1$, X, Y and p have the meanings given under general formula I, and $R_{28}$ is a $C_{1-4}$-alkyl group, under basic conditions and optionally reacting with a compound of general formula XXIII

$R_{27}$ - B (XXIII),

in which $R_{27}$ has the meaning given under general formula I, except a hydrogen atom, and B has the meaning of a halogen atom or the p-toluenesulphonate group, optionally using an acid binding reagent.

## Revendications

**Revendications pour les Etats contractants suivants : AT, BE, CH, DE, FR, GB, GR, IT, LI, LU, NL, SE**

**1.** Azoles et azines avec substituants sur l'hétérocycle, de formule générale I ci-dessous

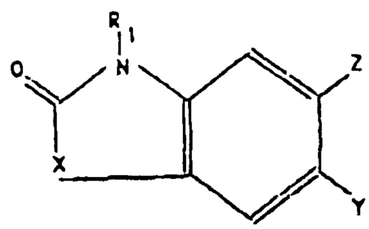

(I),

dans laquelle

| | |
|---|---|
| $R_1$ | représente un atome d'hydrogène, inn alkyle en $C_1$-$C_5$, un alcényle ou un alcynyle en $C_3$-$C_5$, un halogéno-alkyle en $C_1$-$C_4$, inn halogéno-alcényle ou un halogéno-alcynyle en $C_3$-$C_5$ ou encore un cyano-alkyle à alkyle en $C_1$-$C_3$, |
| Y | un atome d'hydrogène ou de fluor, |
| X | un groupe $(CR_2R_3)_n$-W oin $(CR_2)$=V, V et W étant liés directement au cycle phénylique, |
| n | étant le nombre 0 ou 1, |
| W | un groupe $CR_4R_5$ ou $NR_6$ ou encore un atome d'oxygène ou de soufre, avec la condition que si Z est un radical $Z_1$ tel que ci-dessoins et n le nombre 1, W ne soit pas l'oxygène, |
| V | un groupe $CR_4$ ou un atome d'azote, |
| $R_2$, $R_3$, $R_4$ et $R_5$, | qui peuvent être identiques ou différents les uns des autres, étant chacun un atome d'hydrogène ou d'halogène ou bien un alkyle en $C_1$-$C_3$ ou un halogéno-alkyle en $C_1$-$C_3$, et |
| $R_6$ | un atome d'hydrogène, et |
| Z | représente un radical hétérocyclique de l'une des formules $Z_1$ et $Z_3$ à $Z_8$ ci-après |

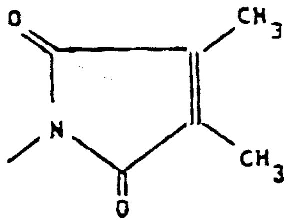

,

$Z_1$

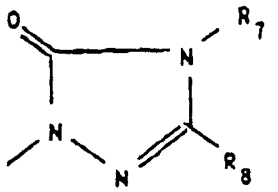

,

$Z_3$

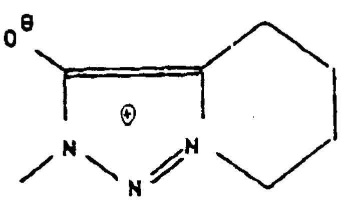

,

$Z_5$

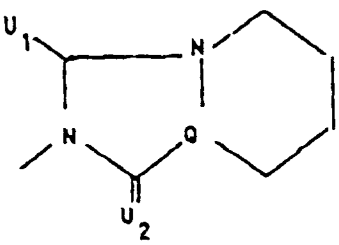

,

$Z_6$

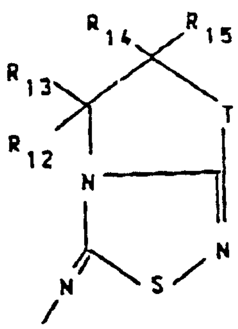

,

$Z_7$

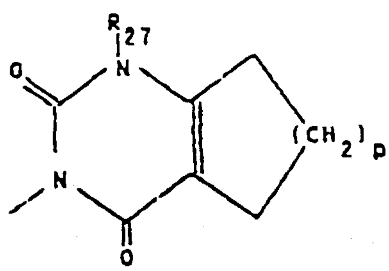

,

$Z_8$

dans lesquelles

| | |
|---|---|
| Q | désigne le groupe CH, avec la condition que si n = 0 W ne soit pas le soufre, |
| $U_1$ | un atome d'oxygène ou de soufre, |
| $U_2$ | un atome d'oxygène, |
| $R_7$ et $R_8$ | forment ensemble et avec les atomes voisins d'azote et de carbone un hétérocycle de 5 à 7 chaînons, saturé ou insaturé, |
| T | désigne un groupe D-E, |
| D | étant un groupe $(CR_{16}R_{17})_m$ et |
| E | un atome de soufre ou un groupe $CR_{19}R_{20}$, |
| m | étant le nombre 0, et |

| | |
|---|---|
| p | = 2, |
| $R_{12}$, $R_{13}$, $R_{14}$, $R_{15}$, $R_{16}$, $R_{17}$, $R_{18}$, $R_{19}$ et $R_{20}$ | sont des atomes d'hydrogène ou des alkyles en $C_1$-$C_4$ et |
| $R_{27}$ | représente un atome d'hydrogène ou un alkyle en $C_1$-$C_5$. |

**2.** Produits herbicides caractérisés en ce qu'ils contiennent un ou plusieurs composés de la revendication 1.

**3.** L'emploi de produits herbicides selon la revendication 2 pour lutter en agriculture contre des mauvaises herbes et plantes adventices monocotylédones et dicotylédones.

**Revendication pour l'Etat contractant suivant : ES**

**1.** Procédé de préparation d'azoles et azines substitués sur l'hétérocycle, de formule générale I ci-dessus

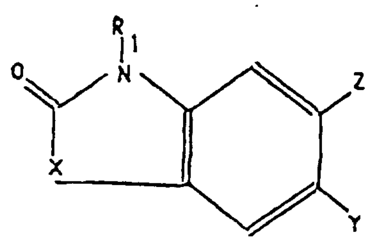

(I),

dans laquelle

| | |
|---|---|
| $R_1$ | représente un atome d'hydrogène, un alkyle en $C_1$-$C_5$, un alcényle ou un alcynyle en $C_3$-$C_5$, un halogéno-alkyle en $C_1$-$C_4$, un halogéno-alcényle ou un halogéno-alcynyle en $C_3$-$C_5$ ou encore un cyano-alkyle à alkyle en $C_1$-$C_3$, |
| Y | un atome d'hydrogène ou de fluor, |
| X | un groupe $(CR_2R_3)_n$-W ou $(CR_2)=V$, V et W étant liés directement au cycle phénylique, |
| n | le nombre 0 ou 1, |
| W | un groupe $CR_4R_5$ ou $NR_6$ ou bien un atome d'oxygène ou de soufre, avec la condition que si le radical Z ci-après est un radical $Z_1$ et n le nombre 1, W ne soit pas l'oxygène, |
| V | représente un groupe $CR_4$ ou un atome d'azote, |
| $R_2$, $R_3$, $R_4$ et $R_5$, | identiques ou différents, sont chacun un atome d'hydrogène ou d'halogène ou bien un alkyle ou un halogéno-alkyle en $C_1$-$C_3$, |
| $R_6$ | est un atome d'hydrogène, |
| Z | un radical hétérocyclique de l'une des formules $Z_1$ et $Z_3$ à $Z_8$ ci-dessous |

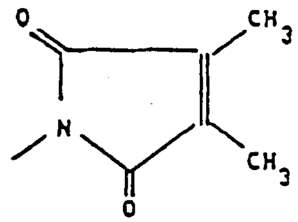

$Z_1$ ,

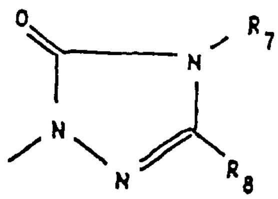

$Z_3$ ,

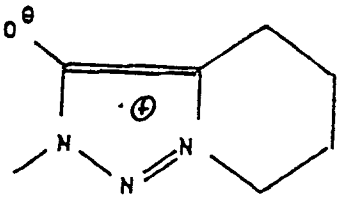

$Z_5$ ,

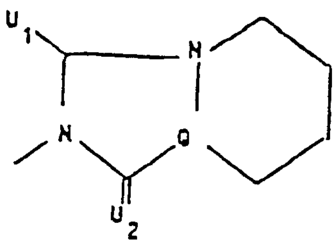

$Z_6$ ,

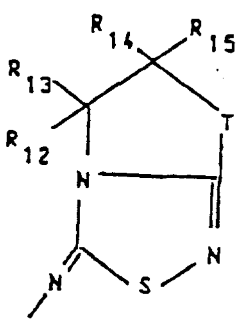

$Z_7$

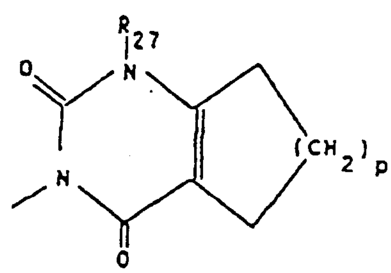

$Z_8$

Q désignant le groupe CH, avec la condition que si n = 0 W ne soit pas le soufre,

$U_1$ un atome d'oxygène ou de soufre,

$U_2$ un atome d'oxygène,

$R_7$ et $R_8$ forment ensemble et avec les atomes d'azote et de carbone voisins un hétérocycle ayant de 5 à 7 chaînons, saturé ou insaturé,

T est un groupe D-E,

| | |
|---|---|
| D | étant un groupe $(CR_{16}R_{17})_m$, |
| E | un atome de soufre ou un groupe $CR_{19}R_{20}$, |
| m | = 0, |
| p | = 2, |
| $R_{12}$, $R_{13}$, $R_{14}$, $R_{15}$, $R_{16}$, $R_{17}$, $R_{18}$, $R_{19}$ et $R_{20}$ | sont des atomes d'hydrogène ou des alkyles en $C_1$-$C_4$, et |
| $R_{27}$ | représente un atome d'hydrogène ou un alkyle en $C_1$-$C_5$, |

procédé caractérisé en ce que :

A) si Z est un radical 3,4-diméthyl-3-pyrroline-2,5-dione-1-yle, on fait réagir une aniline de formule générale II

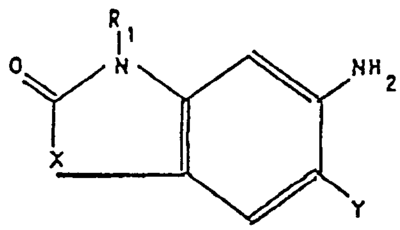

(II),

avec l'anhydride de l'acide 2,2'-diméthylmaléique,

B) si Z est un radical 4,5,6,7-tétrahydro-2H-1,2,3-triazolo-[3,4-a]pyridin-8-ium-3-olate-2-yle, on diazote un composé de formule générale II

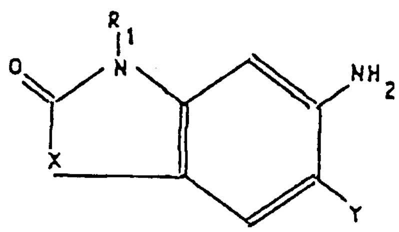

(II),

avec de l'acide nitreux, puis on fait réagir le composé diazoté avec de l'acide pipéridine-2-carboxylique et on cyclise avec des agents déshydratants,

C) si Z est un radical 2,3,5,6,7,8-hexahydro-1H-imidazo[1,5-a]pyridine-1,3-dione-2-yle ou 2,3,5,6,7,8-hexahydro-1H-1,3,4-triazolo[1,2-a]pyridazine-1,3-dione-2-yle, on fait réagir un composé de formule générale II

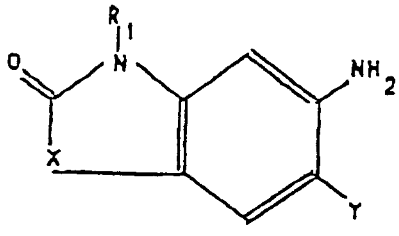

(II),

avec un composé de formule V ou VI

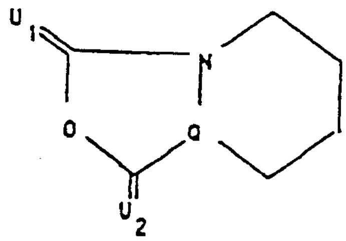 (V) 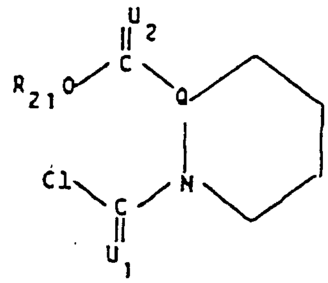 (VI),

$R_{21}$ désignant un alkyle en $C_1$-$C_4$,

D) si Z est un radical 2,3,5,6,7,8-hexahydro-1H-imidazo[1,5-a]pyridine-1,3-dione-2-yle ou 2,3,5,6,7,8-hexahydro-1H-1,3,4-triazolo[1,2-a]pyridazine-1,3-dione-2-yle, on fait réagir un composé de formule générale III

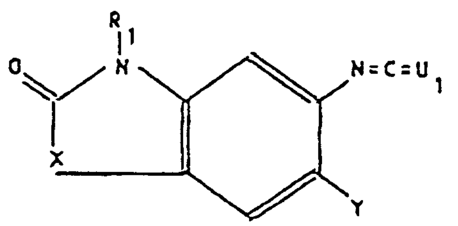

(III),

avec un composé de formule VII

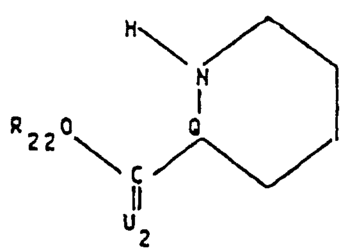

(VII)

$R_{22}$ désignant un alkyle en $C_1$-$C_4$,

E) si Z est un radical

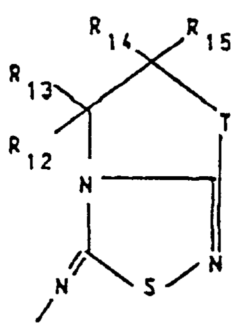

on fait réagir un composé de formule IIIa

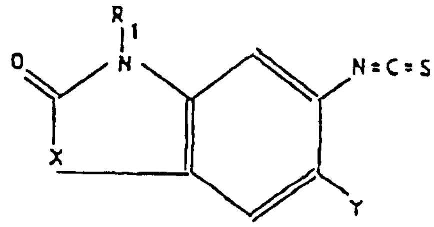

(IIIa),

avec un composé de formule VIII

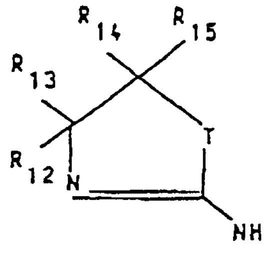

(VIII),

et on cyclise le composé formé en thiadiazole en présence d'un agent d'oxydation,

I) si Z est un radical 2H-1,2,4,-triazolin-3-on-2-yle, on fait réagir un composé de formule générale IV

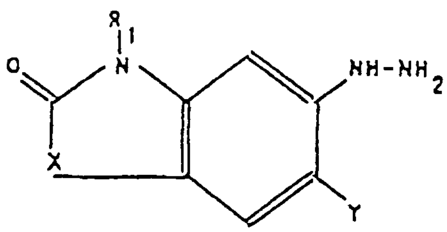

(IV).

avec un composé de formule XI

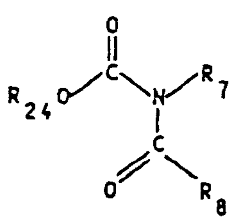

(XI),

$R_{24}$ désignant un alkyle en $C_1$-$C_4$, et enfin

K) si Z est un radical 5,6,7,8-tétrahydroquinazoline-2,4(1H,3H)-dione-3-yle ou 1,2,4,5,6,7-hexahydro-2,4-dioxo-3H-cyclopentapyrimidin-3-yle, on cyclise en milieu basique un composé de formule générale XXII

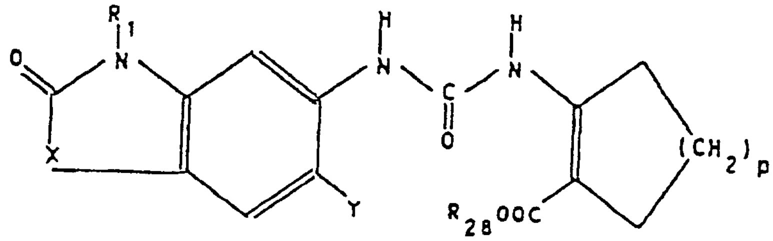

(XXII),

$R_{28}$ désignant un alkyle en $C_1$-$C_4$, et le cas échéant on fait réagir le composé cyclisé avec un composé de formule XXIII

$R_{27}$ - N (XXIII)

$R_{27}$ ayant la signification indiquée pour la formule I à l'exception de l'hydrogène et B désignant un atome d'halogène ou le groupe p-toluène-sulfonyloxy, éventuellement en présence d'un agent liant les acides.